# NEW EUROPEAN PATENT SPECIFICATION

(11) **EP 1 718 731 B2**
(45) Date of publication and mention of the opposition decision: **19.10.2016**
(45) Mention of the grant of the patent: 20.11.2013
(21) Application number: 05710815.1
(22) Date of filing: 25.02.2005
(51) Int. Cl.: C12N 1/21, C12N 9/18, C12P 13/22, C12P 13/04

(54) **Microorganism expressing 6-phosphogluconolactonase and its use in the production of L-amino acids.**
Mikroorganismus welche 6-phosphogluconolactonase exprimiert und dessen Verwendung zur Herstellung von L-Aminosäuren.
Microorganisme exprimant une 6-phosphogluconolactonase et son utilisation dans la production de L-acides aminés.

(30) Priority: 25.02.2004 RU 2004105179; 27.08.2004 US 604698 P; 26.01.2005 RU 2005101700
(43) Date of publication of application: 08.11.2006
(73) Proprietor: Ajinomoto Co., Inc., Tokyo 104-8315 (JP)
(72) Inventor: ZIMENKOV, Danila, Vadimovich, Moscow 105077 (RU); GULEVICH, Andrey, Yurievich, Moscow 109559 (RU); SKOROKHODOVA, Aleksandra, Yurievna, Moscow 115304 (RU); KATASHKINA, Joanna, Yosifovna, Moscow 115304 (RU); KIVERO, Aleksandr, Dmitrievich, Moscow 115404 (RU); BIRYUKOVA, Irina, Vladimirovna, Moscow 115516 (RU); DOROSHENKO, Vera, Georgievna, Moscow 113628 (RU); MASHKO, Sergei, Vladimirovich, Moscow 105043 (RU)
(74) Representative: Strehl Schübel-Hopf & Partner
(86) International application number: PCT/JP2005/003695
(87) International publication number: WO 2005/080583

(56) References cited:
- WO-A-01/07626
- WO-A-99/55877
- WO-A-03/044192
- COLLARD F ET AL: "Identification of the cDNA encoding human 6-phosphogluconolactonase, the enzyme catalyzing the second step of the pentose phosphate pathway" FEBS LETTERS, ELSEVIER SCIENCE PUBLISHERS, AMSTERDAM, NL, vol. 459, no. 2, 8 October 1999 (1999-10-08), pages 223-226, XP004260350 ISSN: 0014-5793 cited in the application
- DUFFIEUX FRANCIS ET AL: "Molecular characterization of the first two enzymes of the pentose-phosphate pathway of Trypanosoma brucei: Glucose-6-phosphate dehydrogenase and 6-phosphogluconolactonase" JOURNAL OF BIOLOGICAL CHEMISTRY, vol. 275, no. 36, 8 September 2000 (2000-09-08), pages 27559-27565, XP002339707 ISSN: 0021-9258 cited in the application
- ZIMENKOV D ET AL: "Escherichia coli ORF ybhE is pgl gene encoding 6-phosphogluconolactonase (EC 3.1.1.31) that has no homology with known 6PGLs from other organisms" FEMS MICROBIOLOGY LETTERS, AMSTERDAM, NL, vol. 244, no. 2, 8 February 2005 (2005-02-08), pages 275-280, XP004781745 ISSN: 0378-1097
- FLORES N ET AL: "PATHWAY ENGINEERING FOR THE PRODUCTION OF AROMATIC COMPOUNDS IN ESCHERICHIA COLI" NATURE BIOTECHNOLOGY, NATURE PUBLISHING, US, vol. 14, May 1996 (1996-05), pages 620-623, XP002053707 ISSN: 1087-0156
- THOMASON LYNN C ET AL: "Identification of the Escherichia coli K-12 ybhE gene as pgl, encoding 6-phosphogluconolactonase", 20041201; 20041200, vol. 186, no. 24, 1 December 2004 (2004-12-01), pages 8248-8253, XP002339708,

### SEQUENCE LISTING

<110> Ajinomoto Co., Inc.
<120> METHOD FOR PRODUCING L-AMINO ACIDS
<130> C2550PC4299
<140>
   <141>
<150> RU2004105179
   <151> 2004-02-25
<150> US60/604,698
   <151> 2004-08-27
<150> RU2005101700
   <151> 2005-01-26
<160> 38
<170> PatentIn Ver. 2.1
<210> 1
   <211> 996
   <212> DNA
   <213> Escherichia coli
<220>
   <221> CDS
   <222> (1)..(996)
<400> 1
   atg aag caa aca gtt tat atc gcc agc cct gag agc cag caa att cac     48
   Met Lys Gln Thr Val Tyr Ile Ala Ser Pro Glu Ser Gln Gln Ile His
    1               5                  10                  15
   gtc tgg aat ctg aat cat gaa ggc gca ctg acg ctg aca cag gtt gtc     96
   Val Trp Asn Leu Asn His Glu Gly Ala Leu Thr Leu Thr Gln Val Val
               20                  25                  30
   gat gtg ccg ggg cag gtg cag ccg atg gtg gtc agc ccg gac aaa cgt    144
   Asp Val Pro Gly Gln Val Gln Pro Met Val Val Ser Pro Asp Lys Arg
           35                  40                  45
   tat ctc tat gtt ggt gtt cgc cct gag ttt cgc gtc ctg gcg tat cgt    192
   Tyr Leu Tyr Val Gly Val Arg Pro Glu Phe Arg Val Leu Ala Tyr Arg
       50                  55                  60
   atc gcc ccg gac gat ggc gca ctg acc ttt gcc gca gag tct gcg ctg    240
   Ile Ala Pro Asp Asp Gly Ala Leu Thr Phe Ala Ala Glu Ser Ala Leu
   65                  70                  75                  80
   ccg ggt agt ccg acg cat att tcc acc gat cac cag ggg cag ttt gtc    288
   Pro Gly Ser Pro Thr His Ile Ser Thr Asp His Gln Gly Gln Phe Val
                   85                  90                  95
   ttt gta ggt tct tac aat gcg ggt aac gtg agc gta acg cgt ctg gaa    336
   Phe Val Gly Ser Tyr Asn Ala Gly Asn Val Ser Val Thr Arg Leu Glu
               100                 105                 110
   gat ggc ctg cca gtg ggc gtc gtc gat gtg gtc gag ggg ctg gac ggt    384
   Asp Gly Leu Pro Val Gly Val Val Asp Val Val Glu Gly Leu Asp Gly
           115                 120                 125
   tgc cat tcc gcc aat atc tca ccg gac aac cgt acg ctg tgg gtt ccg    432
   Cys His Ser Ala Asn Ile Ser Pro Asp Asn Arg Thr Leu Trp Val Pro
       130                 135                 140
   gca tta aag cag gat cgc att tgc ctg ttt acg gtc agc gat gat ggt    480
   Ala Leu Lys Gln Asp Arg Ile Cys Leu Phe Thr Val Ser Asp Asp Gly
   145                 150                 155                 160
   cat ctc gtg gcg cag gac cct gcg gaa gtg acc acc gtt gaa ggg gcc    528
   His Leu Val Ala Gln Asp Pro Ala Glu Val Thr Thr Val Glu Gly Ala
                   165                 170                 175
   ggc ccg cgt cat atg gta ttc cat cca aac gaa caa tat gcg tat tgc    576
   Gly Pro Arg His Met Val Phe His Pro Asn Glu Gln Tyr Ala Tyr Cys
               180                 185                 190
   gtc aat gag tta aac agc tca gtg gat gtc tgg gaa ctg aaa gat ccg    624
   Val Asn Glu Leu Asn Ser Ser Val Asp Val Trp Glu Leu Lys Asp Pro
           195                 200                 205
   cac ggt aat atc gaa tgt gtc cag acg ctg gat atg atg ccg gaa aac    672
   His Gly Asn Ile Glu Cys Val Gln Thr Leu Asp Met Met Pro Glu Asn
       210                 215                 220
   ttc tcc gac acc cgt tgg gcg gct gat att cat atc acc ccg gat ggt    720
   Phe Ser Asp Thr Arg Trp Ala Ala Asp Ile His Ile Thr Pro Asp Gly
   225                 230                 235                 240
   cgc cat tta tac gcc tgc gac cgt acc gcc agc ctg att acc gtt ttc    768
   Arg His Leu Tyr Ala Cys Asp Arg Thr Ala Ser Leu Ile Thr Val Phe
                   245                 250                 255
   agc gtt tcg gaa gat ggc agc gtg ttg agt aaa gaa ggc ttc cag cca    816
   Ser Val Ser Glu Asp Gly Ser Val Leu Ser Lys Glu Gly Phe Gln Pro
               260                 265                 270
   acg gaa acc cag ccg cgc ggc ttc aat gtt gat cac agc ggc aag tat    864
   Thr Glu Thr Gln Pro Arg Gly Phe Asn Val Asp His Ser Gly Lys Tyr
           275                 280                 285
   ctg att gcc gcc ggg caa aaa tct cac cac atc tcg gta tac gaa att    912
   Leu Ile Ala Ala Gly Gln Lys Ser His His Ile Ser Val Tyr Glu Ile
       290                 295                 300
   gtt ggc gag cag ggg cta ctg cat gaa aaa ggc cgc tat gcg gtc ggg    960
   Val Gly Glu Gln Gly Leu Leu His Glu Lys Gly Arg Tyr Ala Val Gly
   305                 310                 315                 320
   cag gga cca atg tgg gtg gtg gtt aac gca cac taa                    996
   Gln Gly Pro Met Trp Val Val Val Asn Ala His
                   325                 330
<210> 2
   <211> 331
   <212> PRT
   <213> Escherichia coli
<400> 2
   Met Lys Gln Thr Val Tyr Ile Ala Ser Pro Glu Ser Gln Gln Ile His
   1               5                   10                  15
   Val Trp Asn Leu Asn His Glu Gly Ala Leu Thr Leu Thr Gln Val Val
               20                  25                  30
   Asp Val Pro Gly Gln Val Gln Pro Met Val Val Ser Pro Asp Lys Arg
           35                  40                  45
   Tyr Leu Tyr Val Gly Val Arg Pro Glu Phe Arg Val Leu Ala Tyr Arg
       50                  55                  60
   Ile Ala Pro Asp Asp Gly Ala Leu Thr Phe Ala Ala Glu Ser Ala Leu
   65                  70                  75                  80
   Pro Gly Ser Pro Thr His Ile Ser Thr Asp His Gln Gly Gln Phe Val
                   85                  90                  95
   Phe Val Gly Ser Tyr Asn Ala Gly Asn Val Ser Val Thr Arg Leu Glu
               100                 105                 110
   Asp Gly Leu Pro Val Gly Val Val Asp Val Val Glu Gly Leu Asp Gly
           115                 120                 125
   Cys His Ser Ala Asn Ile Ser Pro Asp Asn Arg Thr Leu Trp Val Pro
       130                 135                 140
   Ala Leu Lys Gln Asp Arg Ile Cys Leu Phe Thr Val Ser Asp Asp Gly
   145                 150                 155                 160
   His Leu Val Ala Gln Asp Pro Ala Glu Val Thr Thr Val Glu Gly Ala
                   165                 170                 175
   Gly Pro Arg His Met Val Phe His Pro Asn Glu Gln Tyr Ala Tyr Cys
               180                 185                 190
   Val Asn Glu Leu Asn Ser Ser Val Asp Val Trp Glu Leu Lys Asp Pro
           195                 200                 205
   His Gly Asn Ile Glu Cys Val Gln Thr Leu Asp Met Met Pro Glu Asn
       210                 215                 220
   Phe Ser Asp Thr Arg Trp Ala Ala Asp Ile His Ile Thr Pro Asp Gly
   225                 230                 235                 240
   Arg His Leu Tyr Ala Cys Asp Arg Thr Ala Ser Leu Ile Thr Val Phe
                   245                 250                 255
   Ser Val Ser Glu Asp Gly Ser Val Leu Ser Lys Glu Gly Phe Gln Pro
               260                 265                 270
   Thr Glu Thr Gln Pro Arg Gly Phe Asn Val Asp His Ser Gly Lys Tyr
           275                 280                 285
   Leu Ile Ala Ala Gly Gln Lys Ser His His Ile Ser Val Tyr Glu Ile
       290                 295                 300
   Val Gly Glu Gln Gly Leu Leu His Glu Lys Gly Arg Tyr Ala Val Gly
   305                 310                 315                 320
   Gln Gly Pro Met Trp Val Val Val Asn Ala His
                   325                 330
<210> 3
   <211> 58
   <212> DNA
   <213> Artificial Sequence
<220>
   <223> Description of Artificial Sequence: primer P1
<400> 3
   catgaagcaa acagtttata tcgccagccc tgagagctta cgccccgccc tgccactc 58
<210> 4
   <211> 59
   <212> DNA
   <213> Artificial Sequence
<220>
   <223> Description of Artificial Sequence: primer P2
<400> 4
   ttagtgtgcg ttaaccacca cccacattgg tccctggctg atgtccggcg gtgcttttg 59
<210> 5
   <211> 1096
   <212> DNA
   <213> Artificial Sequence
<220>
   <223> Description of Artificial Sequence: 36 nucleotides from 5'-terminus of ybhE gene, Cm-resistance gene, 36 nucleotides from 3'-terminus of ybhE gene
<400> 5
   catgaagcaa acagtttata tcgccagccc tgagagctta cgccccgccc tgccactcat 60
   cgcagtactg ttgtaattca ttaagcattc tgccgacatg gaagccatca cagacggcat 120
   gatgaacctg aatcgccagc ggcatcagca ccttgtcgcc ttgcgtataa tatttgccca 180
   tggtgaaaac gggggcgaag aagttgtcca tattggccac gtttaaatca aaactggtga 240
   aactcaccca gggattggct gagacgaaaa acatattctc aataaaccct ttagggaaat 300
   aggccaggtt ttcaccgtaa cacgccacat cttgcgaata tatgtgtaga aactgccgga 360
   aatcgtcgtg gtattcactc cagagcgatg aaaacgtttc agtttgctca tggaaaacgg 420
   tgtaacaagg gtgaacacta tcccatatca ccagctcacc gtctttcatt gccatacgga 480
   attccggatg agcattcatc aggcgggcaa gaatgtgaat aaaggccgga taaaacttgt 540
   gcttattttt ctttacggtc tttaaaaagg ccgtaatatc cagctgaacg gtctggttat 600
   aggtacattg agcaactgac tgaaatgcct caaaatgttc tttacgatgc cattgggata 660
   tatcaacggt ggtatatcca gtgatttttt tctccatttt agcttcctta gctcctgaaa 720
   atctcgataa ctcaaaaaat acgcccggta gtgatcttat ttcattatgg tgaaagttgg 780
   aacctcttac gtgccgatca acgtctcatt ttcgccaaaa gttggcccag ggcttcccgg 840
   tatcaacagg gacaccagga tttatttatt ctgcgaagtg atcttccgtc acaggtattt 900
   attcggcgca aagtgcgtcg ggtgatgctg ccaacttact gatttagtgt atgatggtgt 960
   ttttgaggtg ctccagtggc ttctgtttct atcagctgtc cctcctgttc agctactgac 1020
   ggggtggtgc gtaacggcaa aagcaccgcc ggacatcagc cagggaccaa tgtgggtggt 1080
   ggttaacgca cactaa 1096
<210> 6
   <211> 25
   <212> DNA
   <213> Artificial Sequence
<220>
   <223> Description of Artificial Sequence: primer P3
<400> 6
   tacaccgata ccactatcgg acaaa 25
<210> 7
   <211> 19
   <212> DNA
   <213> Artificial Sequence
<220>
   <223> Description of Artificial Sequence: primer P4
<400> 7
   gaacgccaga gacacgcgt 19
<210> 8
   <211> 59
   <212> DNA
   <213> Artificial Sequence
<220>
   <223> Description of Artificial Sequence: primer P5
<400> 8
   ttaaatcagg tggctataaa tgaactgggc aatgctgctg atgtccggcg gtgcttttg 59
<210> 9
   <211> 57
   <212> DNA
   <213> Artificial Sequence
<220>
   <223> Description of Artificial Sequence: primer P6
<400> 9
   atgaccacac gcgtgattgc tctcgactta gacggcttac gccccgccct gccactc 57
<210> 10
   <211> 59
   <212> DNA
   <213> Artificial Sequence
<220>
   <223> Description of Artificial Sequence: primer P7
<400> 10
   ttaacctatc tcctgtaacg cgtgtctctg gcgttcgctg atgtccggcg gtgcttttg 59
<210> 11
   <211> 57
   <212> DNA
   <213> Artificial Sequence
<220>
   <223> Description of Artificial Sequence: primer P8
<400> 11
   atgcagttaa aatttttaac ggccagccac ccaaaattac gccccgccct gccactc 57
<210> 12
   <211> 1095
   <212> DNA
   <213> Artificial Sequence
<220>
   <223> Description of Artificial Sequence: 36 nucleotides from 3'-terminus of ybhE gene, Cm-resistance gene, 36 nucleotides from 5'-terminus of ybhA gene
<400> 12
   ttaaatcagg tggctataaa tgaactgggc aatgctgctg atgtccggcg gtgcttttgc 60
   cgttacgcac caccccgtca gtagctgaac aggagggaca gctgatagaa acagaagcca 120
   ctggagcacc tcaaaaacac catcatacac taaatcagta agttggcagc atcacccgac 180
   gcactttgcg ccgaataaat acctgtgacg gaagatcact tcgcagaata aataaatcct 240
   ggtgtccctg ttgataccgg gaagccctgg gccaactttt ggcgaaaatg agacgttgat 300
   cggcacgtaa gaggttccaa ctttcaccat aatgaaataa gatcactacc gggcgtattt 360
   tttgagttat cgagattttc aggagctaag gaagctaaaa tggagaaaaa aatcactgga 420
   tataccaccg ttgatatatc ccaatggcat cgtaaagaac attttgaggc atttcagtca 480
   gttgctcaat gtacctataa ccagaccgtt cagctggata ttacggcctt tttaaagacc 540
   gtaaagaaaa ataagcacaa gttttatccg gcctttattc acattcttgc ccgcctgatg 600
   aatgctcatc cggaattccg tatggcaatg aaagacggtg agctggtgat atgggatagt 660
   gttcaccctt gttacaccgt tttccatgag caaactgaaa cgttttcatc gctctggagt 720
   gaataccacg acgatttccg gcagtttcta cacatatatt cgcaagatgt ggcgtgttac 780
   ggtgaaaacc tggcctattt ccctaaaggg tttattgaga atatgttttt cgtctcagcc 840
   aatccctggg tgagtttcac cagttttgat ttaaacgtgg ccaatatgga caacttcttc 900
   gcccccgttt tcaccatggg caaatattat acgcaaggcg acaaggtgct gatgccgctg 960
   gcgattcagg ttcatcatgc cgtctgtgat ggcttccatg tcggcagaat gcttaatgaa 1020
   ttacaacagt actgcgatga gtggcagggc ggggcgtaag ccgtctaagt cgagagcaat 1080
   cacgcgtgtg gtcat 1095
<210> 13
   <211> 1095
   <212> DNA
   <213> Artificial Sequence
<220>
   <223> Description of Artificial Sequence: 36 nucleotides from 3-terminus of ybhE gene, Cm-resistance gene, 36 nucleotides from 5'-terminus of ybhD gene
<400> 13
   ttaacctatc tcctgtaacg cgtgtctctg gcgttcgctg atgtccggcg gtgcttttgc 60
   cgttacgcac caccccgtca gtagctgaac aggagggaca gctgatagaa acagaagcca 120
   ctggagcacc tcaaaaacac catcatacac taaatcagta agttggcagc atcacccgac 180
   gcactttgcg ccgaataaat acctgtgacg gaagatcact tcgcagaata aataaatcct 240
   ggtgtccctg ttgataccgg gaagccctgg gccaactttt ggcgaaaatg agacgttgat 300
   cggcacgtaa gaggttccaa ctttcaccat aatgaaataa gatcactacc gggcgtattt 360
   tttgagttat cgagattttc aggagctaag gaagctaaaa tggagaaaaa aatcactgga 420
   tataccaccg ttgatatatc ccaatggcat cgtaaagaac attttgaggc atttcagtca 480
   gttgctcaat gtacctataa ccagaccgtt cagctggata ttacggcctt tttaaagacc 540
   gtaaagaaaa ataagcacaa gttttatccg gcctttattc acattcttgc ccgcctgatg 600
   aatgctcatc cggaattccg tatggcaatg aaagacggtg agctggtgat atgggatagt 660
   gttcaccctt gttacaccgt tttccatgag caaactgaaa cgttttcatc gctctggagt 720
   gaataccacg acgatttccg gcagtttcta cacatatatt cgcaagatgt ggcgtgttac 780
   ggtgaaaacc tggcctattt ccctaaaggg tttattgaga atatgttttt cgtctcagcc 840
   aatccctggg tgagtttcac cagttttgat ttaaacgtgg ccaatatgga caacttcttc 900
   gcccccgttt tcaccatggg caaatattat acgcaaggcg acaaggtgct gatgccgctg 960
   gcgattcagg ttcatcatgc cgtctgtgat ggcttccatg tcggcagaat gcttaatgaa 1020
   ttacaacagt actgcgatga gtggcagggc ggggcgtaat tttgggtggc tggccgttaa 1080
   aaattttaac tgcat 1095
<210> 14
   <211> 17
   <212> DNA
   <213> Artificial Sequence
<220>
   <223> Description of Artificial Sequence: primer P9
<400> 14
   cggccaggtg gaagtgg 17
<210> 15
   <211> 16
   <212> DNA
   <213> Artificial Sequence
<220>
   <223> Description of Artificial Sequence: primer P10
<400> 15
   ggctctcagg gctggc 16
<210> 16
   <211> 17
   <212> DNA
   <213> Artificial Sequence
<220>
   <223> Description of Artificial Sequence: primer P11
<400> 16
   cgagcagggg ctactgc 17
<210> 17
   <211> 16
   <212> DNA
   <213> Artificial Sequence
<220>
   <223> Description of Artificial Sequence: primer P12
<400> 17
   aacgcgcccctcgagg 16
<210> 18
   <211> 68
   <212> DNA
   <213> Artificial Sequence
<220>
   <223> Description of Artificial Sequence: primer P13
<400> 18
   ttaaccgcgc cacgctttat agcggttaat cagaccgaaa gccacgttgt gtctcaaaat 60
   ctctgatg 68
<210> 19
   <211> 65
   <212> DNA
   <213> Artificial Sequence
<220>
   <223> Description of Artificial Sequence: primer P14
<400> 19
   aatgaaaaac atcaatccaa cgcagaccgc tgcctggcgc tgaggtctgc ctcgtgaaga 60
   aggtg                                                             65
<210> 20
   <211> 1286
   <212> DNA
   <213> Artificial Sequence
<220>
   <223> Description of Artificial Sequence: 36 nucleotides from 3'-terminus of pgi gene, Km-resistance gene, 36 nucleotides from 5'-terminus of pgi gene
<400> 20
   ttaaccgcgc cacgctttat agcggttaat cagaccgaaa gccacgttgt gtctcaaaat 60
   ctctgatgtt acattgcaca agataaaaat atatcatcat gaacaataaa actgtctgct 120
   tacataaaca gtaatacaag gggtgttatg agccatattc aacgggaaac gtcttgctcg 180
   aggccgcgat taaattccaa catggatgct gatttatatg ggtataaatg ggctcgcgat 240
   aatgtcgggc aatcaggtgc gacaatctat cgattgtatg ggaagcccga tgcgccagag 300
   ttgtttctga aacatggcaa aggtagcgtt gccaatgatg ttacagatga gatggtcaga 360
   ctaaactggc tgacggaatt tatgcctctt ccgaccatca agcattttat ccgtactcct 420
   gatgatgcat ggttactcac cactgcgatc cccgggaaaa cagcattcca ggtattagaa 480
   gaatatcctg attcaggtga aaatattgtt gatgcgctgg cagtgttcct gcgccggttg 540
   cattcgattc ctgtttgtaa ttgtcctttt aacagcgatc gcgtatttcg tctcgctcag 600
   gcgcaatcac gaatgaataa cggtttggtt gatgcgagtg attttgatga cgagcgtaat 660
   ggctggcctg ttgaacaagt ctggaaagaa atgcataagc ttttgccatt ctcaccggat 720
   tcagtcgtca ctcatggtga tttctcactt gataacctta tttttgacga ggggaaatta 780
   ataggttgta ttgatgttgg acgagtcgga atcgcagacc gataccagga tcttgccatc 840
   ctatggaact gcctcggtga gttttctcct tcattacaga aacggctttt tcaaaaatat 900
   ggtattgata atcctgatat gaataaattg cagtttcatt tgatgctcga tgagtttttc 960
   taatcagaat tggttaattg gttgtaacac tggcagagca ttacgctgac ttgacgggac 1020
   ggcggctttg ttgaataaat cgaacttttg ctgagttgaa ggatcagatc acgcatcttc 1080
   ccgacaacgc agaccgttcc gtggcaaagc aaaagttcaa aatcaccaac tggtccacct 1140
   acaacaaagc tctcatcaac cgtggctccc tcactttctg gctggatgat ggggcgattc 1200
   aggcctggta tgagtcagca acaccttctt cacgaggcag acctcagcgc caggcagcgg 1260
   tctgcgttgg attgatgttt ttcatt                                      1286
<210> 21
   <211> 16
   <212> DNA
   <213> Artificial Sequence
<220>
   <223> Description of Artificial Sequence: primer P15
<400> 21
   ttaaccgcgc cacgct 16
<210> 22
   <211> 22
   <212> DNA
   <213> Artificial Sequence
<220>
   <223> Description of Artificial Sequence: primer P16
<400> 22
   aatgaaaaac atcaatccaa cg 22
<210> 23
   <211> 47
   <212> DNA
   <213> Artificial Sequence
<220>
   <223> Description of Artificial Sequence: primer P17
<400> 23
   gacaaagagc tccacacagg aaacagctat gggagggcgt ggtatgg 47
<210> 24
   <211> 33
   <212> DNA
   <213> Artificial Sequence
<220>
   <223> Description of Artificial Sequence: primer P18
<400> 24
   ttagtagaat tctcatgggc accagtagat gtc 33
<210> 25
   <211> 68
   <212> DNA
   <213> Artificial Sequence
<220>
   <223> Description of Artificial Sequence: primer P19
<400> 25
   ttacagctta gcgccttcta cagcttcacg cgccaggaaa gccacgttgt gtctcaaaat 60
   ctctgatg 68
<210> 26
   <211> 65
   <212> DNA
   <213> Artificial Sequence
<220>
   <223> Description of Artificial Sequence: primer P20
<400> 26
   atggcggtaa cgcaaacagc ccaggcctgt gacctggcgc tgaggtctgc ctcgtgaaga 60
   aggtg 65
<210> 27
   <211> 1286
   <212> DNA
   <213> Artificial Sequence
<220>
   <223> Description of Artificial Sequence: 36 nucleotides from 3-terminus of eda gene, Km-resistance gene, 36 nucleotides from 5'-terminus of zwf gene
<400> 27
   ttacagctta gcgccttcta cagcttcacg cgccaggaaa gccacgttgt gtctcaaaat 60
   ctctgatgtt acattgcaca agataaaaat atatcatcat gaacaataaa actgtctgct 120
   tacataaaca gtaatacaag gggtgttatg agccatattc aacgggaaac gtcttgctcg 180
   aggccgcgat taaattccaa catggatgct gatttatatg ggtataaatg ggctcgcgat 240
   aatgtcgggc aatcaggtgc gacaatctat cgattgtatg ggaagcccga tgcgccagag 300
   ttgtttctga aacatggcaa aggtagcgtt gccaatgatg ttacagatga gatggtcaga 360
   ctaaactggc tgacggaatt tatgcctctt ccgaccatca agcattttat ccgtactcct 420
   gatgatgcat ggttactcac cactgcgatc cccgggaaaa cagcattcca ggtattagaa 480
   gaatatcctg attcaggtga aaatattgtt gatgcgctgg cagtgttcct gcgccggttg 540
   cattcgattc ctgtttgtaa ttgtcctttt aacagcgatc gcgtatttcg tctcgctcag 600
   gcgcaatcac gaatgaataa cggtttggtt gatgcgagtg attttgatga cgagcgtaat 660
   ggctggcctg ttgaacaagt ctggaaagaa atgcataagc ttttgccatt ctcaccggat 720
   tcagtcgtca ctcatggtga tttctcactt gataacctta tttttgacga ggggaaatta 780
   ataggttgta ttgatgttgg acgagtcgga atcgcagacc gataccagga tcttgccatc 840
   ctatggaact gcctcggtga gttttctcct tcattacaga aacggctttt tcaaaaatat 900
   ggtattgata atcctgatat gaataaattg cagtttcatt tgatgctcga tgagtttttc 960
   taatcagaat tggttaattg gttgtaacac tggcagagca ttacgctgac ttgacgggac 1020
   ggcggctttg ttgaataaat cgaacttttg ctgagttgaa ggatcagatc acgcatcttc 1080
   ccgacaacgc agaccgttcc gtggcaaagc aaaagttcaa aatcaccaac tggtccacct 1140
   acaacaaagc tctcatcaac cgtggctccc tcactttctg gctggatgat ggggcgattc 1200
   aggcctggta tgagtcagca acaccttctt cacgaggcag acctcagcgc caggtcacag 1260
   gcctgggctg tttgcgttac cgccat                                     1286
<210> 28
   <211> 23
   <212> DNA
   <213> Artificial Sequence
<220>
   <223> Description of Artificial Sequence: primer P21
<400> 28
   ttacagctta gcgccttcta cag 23
<210> 29
   <211> 18
   <212> DNA
   <213> Artificial Sequence
<220>
   <223> Description of Artificial Sequence: primer P22
<400> 29
   catggcggta acgcaaac 18
<210> 30
   <211> 35
   <212> DNA
   <213> Artificial Sequence
<220>
   <223> Description of Artificial Sequence: primer P23
<400> 30
   ctagtaagat ctccctgttt gcaattaatc atcgg 35
<210> 31
   <211> 35
   <212> DNA
   <213> Artificial Sequence
<220>
   <223> Description of Artificial Sequence: primer P24
<400> 31
   ctagtaagat ctccctgttg acaattaatc atcgg 35
<210> 32
   <211> 59
   <212> DNA
   <213> Artificial Sequence
<220>
   <223> Description of Artificial Sequence: primer P25
<400> 32
   tggcgatata aactgtttgc ttcatgaatg ctcctttcct gtgtgaaatt g ttatccgc 59
<210> 33
   <211> 35
   <212> DNA
   <213> Artificial Sequence
<220>
   <223> Description of Artificial Sequence: primer P26
<400> 33
   ctagtaagat ctgctgatgt ccggcggtgc ttttg 35
<210> 34
   <211> 67
   <212> DNA
   <213> Artificial Sequence
<220>
   <223> Description of Artificial Sequence: primer P27
<400> 34
   gccaaaagcg actaatttta gctgttacag tcagttgcta aatgcattac gccccgccct 60
   gccactc 67
<210> 35
   <211> 1099
   <212> DNA
   <213> Artificial Sequence
<220>
   <223> Description of Artificial Sequence: Cm-resistance gene, Ptac-3900 promoter
<400> 35
   ttacgccccg ccctgccact catcgcagta ctgttgtaat tcattaagca ttctgccgac 60
   atggaagcca tcacagacgg catgatgaac ctgaatcgcc agcggcatca gcaccttgtc 120
   gccttgcgta taatatttgc ccatggtgaa aacgggggcg aagaagttgt ccatattggc 180
   cacgtttaaa tcaaaactgg tgaaactcac ccagggattg gctgagacga aaaacatatt 240
   ctcaataaac cctttaggga aataggccag gttttcaccg taacacgcca catcttgcga 300
   atatatgtgt agaaactgcc ggaaatcgtc gtggtattca ctccagagcg atgaaaacgt 360
   ttcagtttgc tcatggaaaa cggtgtaaca agggtgaaca ctatcccata tcaccagctc 420
   accgtctttc attgccatac ggaattccgg atgagcattc atcaggcggg caagaatgtg 480
   aataaaggcc ggataaaact tgtgcttatt tttctttacg gtctttaaaa aggccgtaat 540
   atccagctga acggtctggt tataggtaca ttgagcaact gactgaaatg cctcaaaatg 600
   ttctttacga tgccattggg atatatcaac ggtggtatat ccagtgattt ttttctccat 660
   tttagcttcc ttagctcctg aaaatctcga taactcaaaa aatacgcccg gtagtgatct 720
   tatttcatta tggtgaaagt tggaacctct tacgtgccga tcaacgtctc attttcgcca 780
   aaagttggcc cagggcttcc cggtatcaac agggacacca ggatttattt attctgcgaa 840
   gtgatcttcc gtcacaggta tttattcggc gcaaagtgcg tcgggtgatg ctgccaactt 900
   actgatttag tgtatgatgg tgtttttgag gtgctccagt ggcttctgtt tctatcagct 960
   gtccctcctg ttcagctact gacggggtgg tgcgtaacgg caaaagcacc gccggacatc 1020
   agcagatctc cctgtttgca attaatcatc ggctcgtata atgtgtggaa ttgtgagcgg 1080
   ataacaattt cacacagga 1099
<210> 36
   <211> 1099
   <212> DNA
   <213> Artificial Sequence
<220>
   <223> Description of Artificial Sequence: Cm-resistance gene, Ptac-10000 promoter
<400> 36
   ttacgccccg ccctgccact catcgcagta ctgttgtaat tcattaagca ttctgccgac 60
   atggaagcca tcacagacgg catgatgaac ctgaatcgcc agcggcatca gcaccttgtc 120
   gccttgcgta taatatttgc ccatggtgaa aacgggggcg aagaagttgt ccatattggc 180
   cacgtttaaa tcaaaactgg tgaaactcac ccagggattg gctgagacga aaaacatatt 240
   ctcaataaac cctttaggga aataggccag gttttcaccg taacacgcca catcttgcga 300
   atatatgtgt agaaactgcc ggaaatcgtc gtggtattca ctccagagcg atgaaaacgt 360
   ttcagtttgc tcatggaaaa cggtgtaaca agggtgaaca ctatcccata tcaccagctc 420
   accgtctttc attgccatac ggaattccgg atgagcattc atcaggcggg caagaatgtg 480
   aataaaggcc ggataaaact tgtgcttatt tttctttacg gtctttaaaa aggccgtaat 540
   atccagctga acggtctggt tataggtaca ttgagcaact gactgaaatg cctcaaaatg 600
   ttctttacga tgccattggg atatatcaac ggtggtatat ccagtgattt ttttctccat 660
   tttagcttcc ttagctcctg aaaatctcga taactcaaaa aatacgcccg gtagtgatct 720
   tatttcatta tggtgaaagt tggaacctct tacgtgccga tcaacgtctc attttcgcca 780
   aaagttggcc cagggcttcc cggtatcaac agggacacca ggatttattt attctgcgaa 840
   gtgatcttcc gtcacaggta tttattcggc gcaaagtgcg tcgggtgatg ctgccaactt 900
   actgatttag tgtatgatgg tgtttttgag gtgctccagt ggcttctgtt tctatcagct 960
   gtccctcctg ttcagctact gacggggtgg tgcgtaacgg caaaagcacc gccggacatc 1020
   agcagatctc cctgttgaca attaatcatc ggctcgtata atgtgtggaa ttgtgagcgg 1080
   ataacaattt cacacagga 1099
<210> 37
   <211> 53
   <212> DNA
   <213> Artificial Sequence
<220>
   <223> Description of Artificial Sequence: primer P28
<400> 37
   gatatacata tgcaccacca ccaccaccac aagcaaacag tttatatcgc cag 53
<210> 38
   <211> 33
   <212> DNA
   <213> Artificial Sequence
<220>
   <223> Description of Artificial Sequence: primer P29
<400> 38
   agactaggat ccttagtgtg cgttaaccac cac 33

## Description

### Technical field

The present invention relates to a method for producing L-amino acids by fermentation using a microorganism. Specifically, the present invention relates to a method for producing aromatic ammo acids such as L-tryptophan, L-phenylalanine, and L-tyrosine.

### Background art

The pentose phosphate pathway (PPP) is an important part of the central metabolism of a majority of organisms, In the oxidative branch of PPP, the synthesis of NADPH takes place, and phosphorylated carbohydrates of the non-oxidative branch of PPP are precursors for nucleotide biosynthesis (ribose-5-phosphate), aromatic amino acids and vitamins (erythrose-5-phosphate). Erythrose 4-phosphate (E4p) are the essential precursors of the common biosynthetic pathway for aromatic L-amino acids. Optimization of the specific pathways of phosphoenolpyruvate (PEP) and E4p biosynthesis can, therefore, improve production of aromatic L-amino acids.

The oxidative branch of PPP includes three reactions. The first and third reactions are catalyzed by the well-known enzymes glucose-6-phosphate dehydrogenase (EC 1.1.1.49) and gluconate-6-phosphate dehydrogenase (EC 1.1:1.44), which are encoded by the *zwf* and *gnd* genes, respectively. The second reaction is the hydrolysis of 6-phosphogluconolactone to 6-phosphogluconate (Escherichia coli and Salmonella, Second Edition, Editor in Chief. F.C.Neidhardt ASM Press, Washington D.C., 1996). An enzyme which catalyzes this reaction has been detected in several organisms including, for example, human (Collard, F., et al, FEBS Lett., 459:2, 223-6 (1999)), *Trypanosoma brucei* (Duffieux, F., et al, J. Biol. Chem., 275:36, 27559-65 (2000)), *Plasmodium berghei* (Clarke, J.L, et al, Eur. J. Biochem. 268:7, 2013-9 (2001)), *Pseudomonas aeroginosa* (Hager P.W. et al, J. Bacteriol.,182:14, 3934-41 (2000)), *Pseudomonas putida* (Petruschka, L., et al, FEMS Microbiol. Lett., 215:1, 89-95 (2002)), however it is also known that the reaction can go spontaneously.

δ-6-Phosphogluconolactone, one of the products of the reaction catalyzed by glucose-6-phosphate dehydrogenase, is able to isomerize to γ-6-phosphogluconolactone in the course of intermolecular rearrangement. Only δ-6-phosphogluconolactone is able to hydrolyze to 6-phosphogluconate spontaneously, and exactly that reaction is catalyzed by known 6-phosphogluconolactonases (EC 3.1.1.31) (Miclet E. et al., J Biol Chem., 276:37, 34540-46 (2001)). The *pgl* gene from *E. coli,* presumably encoding 6-phosphogluconolactonase, was mapped on the chromosome of *E. coli* between *att-*λ and *chlD* gene (in the modem databases - *modC* gene). Mutants of *E. coli* (*pgl*⁻) exhibit "maltose-blue" phenotype (Kupor, S.R. and Fraenkel, D.G., J.Bacteriol., 100:3, 1296-1301 (1969)) which is a distinctive feature of strains which accumulate maltodextrine (Adhya S. and Schwartz M., J Bacteriol, 108:2, 621-626 (1971)).

But at present time, neither the sequence nor the exact location of the *pgl* gene on the chromosome of *E. coli* is known. Enzymes having the activity of 6-phosphogluconolactonase from *E. coli* have not been isolated and there are no reports linking enhancement of 6-phosphogluconolactonase activity in the cell of a L-amino acid-producing bacterium with an increase in L-amino acid production.

### Disclosure of the invention

An object of the present invention is to provide a method for producing L-amino acids.

This object was achieved by identifying the fact that *ybhE* open reading frame (ORF) of *E. coli* strain K-12 encodes 6-phosphogluconolactonase and enhanced expression of the *ybhE* ORF (*pgl* gene) can enhance L-amino acid production by the respective L-amino acid producing strains. Thus, the present invention has been completed.

The present invention provides the following.
(1) A method for producing an L-amino acid comprising cultivating an L-amino acid-producing bacterium belonging to the Enterobacteriaceae family in a culture medium, and collecting said L-amino acid from said culture medium, wherein the bacterium has been modified to increase expression of a gene encoding 6-phosphogluconolactonase, wherein the 6-phosphogluconolactonase is a protein selected from the group consisting of:
   (A) a protein comprising the amino acid sequence shown in SEQ ID NO:2; and
   (B) a protein comprising an amino acid sequence which includes deletion, substitution, insertion or addition of one to 20 amino acids in the amino acid sequence shown in SEQ ID NO:2, and which has an activity of 6-phosphogluconolactonase,
   wherein said expression of the gene is increased by increasing a copy number of said gene, by modifying an expression regulatory sequence of said gene, by substituting a native promoter of said gene with a more potent promoter, or by substituting a native Shine-Dalgarno (SD) sequence of said gene with a more efficient SD sequence.
(2) The method according to (1) above, wherein said 6-phosphogluconolactonase gene is selected from the group consisting of:
   (a) a DNA comprising a nucleotide sequence of the nucleotides 1 to 993 in SEQ ID NO:1; and
   (b) a DNA which is hybridizable with a nucleotide sequence of the nucleotides 1 to 993 in SEQ ID NO: 1 under stringent conditions and encodes a protein having an activity of 6-phosphogluconolactonase, and wherein said stringent conditions comprise washing for 15 minutes at 60°C at a salt concentration corresponding to 1 x SSC and 0.1% SDS.
(3) The method according to (1) or (2) above, wherein the bacterium is selected from the genus selected from the group consisting of *Escherichia, Enterobacter, Erwinia, Klebsiella, Pantoea, Providencia, Salmonella, Serratia, Shigella,* and *Morganella.*
(4) The method according to any one of (1) to (3) above, wherein said bacterium is further modified to have enhanced expression of a *yddG* open reading frame, wherein said expression of the *yddG* gene is enhanced by amplifying said gene on a multi-copy vector.
(5) The method according to any one of (1) to (4) above, wherein said L-amino acid is an aromatic L-amino acid selected from the group consisting of L-tryptophan, L-phenylalanine, and L-tyrosine.

### Brief Description of Drawings

Figure 1 shows the structure of bacterial native DNA region around *ybhE* ORF.
Figure 2 shows the structure of bacterial DNA region with deleted *ybhE* ORF.
Figure 3 shows the structure of bacterial DNA region with deleted *ybhA* ORF.
Figure 4 shows the structure of bacterial DNA region with deleted *ybhD* ORF.
Figure 5 shows the structure of bacterial DNA region with deleted *pgi* gene.
Figure 6 shows the structure of bacterial DNA region with deleted *zwf-edd-eda* operon.
Figure 7 shows the structure of bacterial DNA region with artificial promoter region (P*_{tac}**) upstream of *pgl* gene (*ybhE* ORF).
Figure 8 shows the gel separation of (His)6-YbhE protein and purification (photographs). A. Crude extracts of BL21(DE3)[pET-HTybhE] strain. Lines 1, 2, 9 - Protein Molecular Weight Marker; lines 3, 4 - total cell protein of the strain without and with IPTG induction; lines 5, 6 - soluble fraction of the strain without and with IPTG induction; lines 7, 8 - insoluble fraction of the strain without and with IPTG induction. B. Line 1 - total cell proteins of BL21(DE3)[pET-HTybhE]; lines 2, 3, 4, 6 - increasing concentration of the purified (His)6-YbhE; line 5 - Protein Molecular Weight Marker.

### Description of the preferred embodiments

An L-amino acid producing bacterium is described, wherein the bacterium has been modified to enhance an activity of 6-phosphogluconolactonase. In the present invention, the activity of 6-phosphogluconolactonase is enhanced as defined in the claims.
The term "activity of 6-phosphogluconolactonase" means an activity to catalyze the hydrolysis reaction of 6-phosphogluconolacton to 6-phosphogluconate. Activity of 6-phosphogluconolactonase is measured by the method described by, for example, Kupor, S.R. and Fraenkel, D.G. (J. Bacteriol., 100:3, 1296-1301 (1969)).The gene encoding 6-Phosphogluconolactonase may be the *ybhE* gene of *Escherichia coli* or a homologue thereof.

As the gene encoding 6-phosphogluconolactonase of *Escherichia coli* (EC number 3.1.1.31), *pgl* gene including *ybhE* ORF is stated (nucleotide numbers 797809 to 798804 in the sequence of GenBank accession NC_000913.1, gi:16128735). The *ybhE* ORF is located between *ybhA* and *ybhD* ORFs on the chromosome of *E. coli* strain K12. Therefore, *pgl* gene can be obtained by PCR (polymerase chain reaction; refer to White, T.J. et al., Trends Genet., 5, 185 (1989)) utilizing primers prepared based on the nucleotide sequence of the gene.

The *pgl* gene from *Escherichia coli* is exemplified by a DNA which comprises the following DNA (a) or (b):
(a) a DNA which comprises a nucleotide sequence of the nucleotides 1 to 993 in SEQ ID NO: 1; or
(b) a DNA which is hybridizable with a nucleotide sequence of the nucleotides 1 to 993 in SEQ ID NO:1 under stringent conditions and codes for a protein having an activity of 6-phosphogluconolactonase.

The DNA encoding proteins used in the present invention includes a DNA encoding the protein which includes deletion, substitution, insertion or addition of one to 20 amino acids in one or more positions on the protein (A) as long as they do not lose the activity of the protein.

The protein used in the present invention, having the above-described deletions, substitutions, insertions, or additions of one to 20 amino acids, is at least 70% homologous to the protein of SEQ ID NO:2. Percent homology of the protein is measured by comparing the variant sequence to that in SEQ ID NO:2 over the length of the entire sequence and determining the number of like residues. The protein used in the present invention is at least 70% homologous to the protein of SEQ ID NO:2, more preferably at least 80% homologous, even more preferably at least 90% homologous, and most preferably at least 95% homologous to the protein of SEQ ID NO:2. Percent homology of a protein or DNA can also be evaluated by known calculation methods such as BLAST search, FASTA search and CrustalW. BLAST (Basic Local Alignment Search Tool) is the heuristic search algorithm employed by the programs blastp, blastn, blastx, megablast, tblastn, and tblastx; these programs ascribe significance to their findings using the statistical methods of Karlin, Samuel and Stephen F. Altschul ("Methods for assessing the statistical significance of molecular sequence features by using general scoring schemes". Proc. Natl. Acad. Sci. USA, 1990, 87:2264-68; "Applications and statistics for multiple high-scoring segments in molecular sequences". Proc. Natl. Acad. Sci. USA, 1993, 90:5873-7). FASTA search method described by W. R. Pearson ("Rapid and Sensitive Sequence Comparison with FASTP and FASTA", Methods in Enzymology, 1990 183:63- 98). ClustalW method described by Thompson J.D., Higgins D.G. and Gibson T.J. ("CLUSTAL W: improving the sensitivity of progressive multiple sequence alignment through sequence weighting, position-specific gap penalties and weight matrix choice", Nucleic Acids Res. 1994, 22:4673-4680).

Changes to the protein defined in (A) such as those described above are typically conservative changes so as to maintain the activity of the protein. Substitution changes include those in which at least one residue in the amino acid sequence has been removed and a different residue inserted in its place. Examples of amino acids which may be substituted for an original amino acid in the above protein and which are regarded as conservative substitutions include: Ala substituted with ser or thr; arg substituted with gln, his, or lys; asn substituted with glu, gln, lys, his, asp; asp substituted with asn, glu, or gln; cys substituted with ser or ala; gln substituted with asn, glu, lys, his, asp, or arg; glu substituted with asn, gln, lys, or asp; gly substituted with pro; his substituted with asn, lys, gln, arg, tyr; ile substituted with leu, met, val, phe; leu substituted with ile, met, val, phe; lys substituted with asn, glu, gln, his, arg; met substituted with ile, leu, val, phe; phe substituted with trp, tyr, met, ile, or leu; ser substituted with thr, ala; thr substituted with ser or ala; trp substituted with phe, tyr; tyr substituted with his, phe, or trp; and val substituted with met, ile, leu.

The DNA encoding substantially the same protein as the protein defined in (A) may be obtained by, for example, modification of nucleotide sequence encoding the protein defined in (A) using site-directed mutagenesis so that one or more amino acid residue will be deleted, substituted, inserted, or added. Such modified DNA can be obtained by conventional methods using treatment with reagents and conditions generating mutations. Such treatment includes treatment the DNA encoding proteins used in present invention with hydroxylamin or treatment the bacterium harboring the DNA with UV irradiation or reagent such as N-methyl-N'-nitro-N-nitrosoguanidine or nitrous acid.

The DNA encoding proteins used in the present invention includes variants which can be found in the different strains and variants of bacteria belonging to the genus *Escherichia* according to natural diversity. The DNA encoding such variants can be obtained by isolating the DNA, which hybridizes with *pgl* gene or part of the gene under the stringent conditions, and which encodes the protein having activity of 6-phosphogluconolactonase. The term "stringent conditions" referred to herein is a condition under which a so-called specific hybrid is formed, and a non-specific hybrid is not formed.
Stringent conditions are conditions which comprise ordinary conditions of washing in Southern hybridization, i.e. 60°C, 15 minutes x SSC, 0.1 % SDS, preferably 0.1 x SSC, 0.1% SDS. Duration of washing depends on the type of membrane used for blotting and, as a rule, is recommended by the manufacturer. For example, recommended duration of washing of the Hybond™ N+ nylon membrane (Amersham) under stringent conditions is 15 minutes. Preferably, washing may be performed 2 to 3 times.

Transformation of a bacterium with a DNA encoding a protein means introduction of the DNA into bacterium cell for example by conventional methods to increase expression of the gene encoding the protein used in the present invention and to enhance the activity of the protein in the bacterial cell.

A bacterium used in the present invention is L-amino acid-producing bacterium belonging to the *Enterobacteriaceae* family having enhanced activities of a protein, which enhances the productivity of the target L-amino acid. Preferably, the bacterium used in the present invention is an aromatic L-amino acid-producing bacterium specifically belonging to the genus *Escherichia* that has enhanced activity of the protein used in the present invention. More preferably, the bacterium used in the present invention is an aromatic L-amino acid-producing bacterium, such as L-tryptophan-producing bacterium, specifically belonging to the genus *Escherichia,* wherein the bacterium has been modified to enhance an activity of 6-phosphogluconolactonase. More preferably, the bacterium used in the present invention harbors the DNA comprising the *pgl* gene (*ybhE* ORF) with a modified expression control sequence on the chromosome of the bacterium and has enhanced ability to produce L-tryptophan.

"L-amino acid-producing bacterium" means a bacterium, which has an ability to cause accumulation of the L-amino acid in a medium when the bacterium used in the present invention is cultured in the medium. The L-amino acid-producing ability may be imparted or enhanced by breeding. The term "L-amino acid-producing bacterium" used herein also means a bacterium, which is able to produce and cause accumulation of a L-amino acid in a culture medium in amount larger than a wild-type or parental strain, and preferably means that the microorganism is able to produce and cause accumulation in a medium of an amount not less than 0.5 g/L, more preferably not less than 1.0 g/L of target L-amino acid. L-amino acids include L-alanine, L-arginine, L-asparagine, L-aspartic acid, L-cysteine, L-glutamic acid, L-glutamine, L-glycine, L-histidine, L-isoleucine, L-leucine, L-lysine, L-methionine, L-phenylalanine, L-proline, L-serine, L-threonine, L-tryptophan, L-tyrosine, and L-valine, and preferably includes aromatic L-amino acids, such as L-tryptophan, L-phenylalanine, and L-tyrosine.

The *Enterobacteriaceae* family includes bacteria belonging to the genera *Escherichia, Enterobacter, Erwinia, Klebsiella, Pantoea, Providencia, Salmonella, Serratia, Shigella, Morganella.Enterobacter, Erwinia, Escherichia, Klebsiella, Providencia, Salmonella, Serratia, Shigella* etc. Specifically, those classified into the *Enterobacteriaceae* according to the taxonomy used in the NCBI (National Center for Biotechnology Information) database (http://www.ncbi.nlm.nih.gov/htbinpost/Taxonomy/wgetorg?mode=Tree&id=1236&lvl=3&ke ep=1&srchmode=1&unlock) can be used. The genus *Escherichia* is preferred.

The phrase "a bacterium belonging to the genus *Escherichia*" means that the bacterium is classified in the genus *Escherichia* according to the classification known to a person skilled in the art of microbiology. Examples of a microorganism belonging to the genus *Escherichia* as used in the present invention include, but are not limited to, *Escherichia coli* (*E. coli*).

The bacterium belonging to the genus *Escherichia* that can be used in the present invention is not particularly limited, however for example, bacteria described by Neidhardt, F.C. et al. (Escherichia coli and Salmonella typhimurium, American Society for Microbiology, Washington D.C., 1208, Table 1) are encompassed. Examples of wild-type strains of *Escherichia coli* include, but are not limited to, the K12 strain and derivatives thereof, *Escherichia coli* MG1655 strain (ATCC No. 47076), and W3110 strain (ATCC No. 27325). These strains are available from the American Type Culture Collection (ATCC, Address: 12301 Parklawn Drive, Rockville Maryland 20852, United States of America).

The term "a bacterium belonging to the genus *Pantoea*" means that the bacterium is classified as the genus *Pantoea* according to the classification known to a person skilled in the art of microbiology. Some species of *Enterobacter agglomerans* have been recently re-classified into *Pantoea agglomerans, Pantoea ananatis, Pantoea stewartii* or the like, based on nucleotide sequence analysis of 16S rRNA etc.

The term "modified to enhance an activity of 6-phosphogluconolactonase" means that the activity per cell has become higher than that of a non-modified strain, for example, a wild-type strain. The activity of 6-phosphogluconolactonase can be measured by using Collard's method (FEBS Letters 459 (1999) 223-226). An example is when the number of 6-phosphogluconolactonase molecules per cell increases, the specific activity per 6-phosphogluconolactonase molecule increases and so forth. Furthermore, as a wild-type strain that serves as an object for comparison, for example, the *Escherichia coli* K-12 is encompassed. As a result of enhanced intracellular activity of 6-phosphogluconolactonase, the amount of L-amino acid such as L-tryptophan, which is accumulates in a medium, is increased.

In the present invention, enhancement of 6-phosphogluconolactonase activity in a bacterial cell is attained by increasing expression of a gene encoding 6-phosphogluconolactonase. (*pgl* gene) As the 6-phosphogluconolactonase genes, a gene derived from a bacterium from *Enterobacteriaceae* is encompassed. Expression of *the pgl* gene can be enhanced by, for example, increasing the copy number of the *pgl* gene in cells using genetic recombination techniques. For example, a recombinant DNA can be prepared by ligating a gene fragment containing the *pgl* gene to a vector, preferably a multi-copy vector, which is operable in cells of a host microorganism, and introducing the resulting vector into the cells of the host microorganism.

When the *pgl* gene of *Escherichia coli* is used, *the pgl* gene (*ybhE*) may be obtained by, for example, the PCR method (polymerase chain reaction, refer to White, T.J. et al., Trends Gene., 5, 185 (1989)) using primers designed based on a nucleotide sequence of SEQ ID NO: 1, using chromosomal DNA of *Escherichia coli* as a template. The *pgl* gene from other microorganisms may also be used, and can be obtained from their chromosomal DNA or chromosomal DNA library by PCR using oligonucleotide primers designed based on a sequence of their *pgl* gene or a homologous sequence thereof of *pgl* gene or the 6-phosphogluconolactonase protein from a different species of microorganisms, or by hybridization using an oligonucleotide probe prepared based on such sequence information. A chromosomal DNA can be prepared from a microorganism serving as a DNA donor by, for example, the method of Saito and Miura (refer to H. Saito and K. Miura, Biochem. Biophys. Acta, 72, 619 (1963), Text for Bioengineering Experiments, Edited by the Society for Bioscience and Bioengineering, Japan, pp.97-98, Baifukan, 1992).

Then, the *pgl* gene is ligated to a vector DNA operable in cells of the host microorganism to prepare a recombinant DNA. Preferably, vectors autonomously replicable in cells of the host microorganism are used.

Examples of vectors autonomously replicable in *Escherichia coli* include pUC19, pUC18, pHSG299, pHSG399, pHSG398, pACYC184, (pHSG and pACYC are available from Takara Bio), RSF1010, pBR322, pMW219 (pMW is available from Nippon Gene), and so forth.

In order to prepare a recombinant DNA by ligating the *pgl* gene and any of the vectors mentioned above, the vector and a fragment containing the *pgl* gene are digested with restriction enzymes and ligated with each other, usually by using a ligase such as a T4 DNA ligase.

To introduce a recombinant DNA prepared as described above into a microorganism, any known transformation methods reported so far can be employed. For example, a method of treating recipient cells with calcium chloride so as to increase the permeability of DNA, which has been reported for *Escherichia coli* (Mandel, M. and Higa, A., J. Mol. Biol., 53,159 (1970)), and a method of using competent cells prepared from growing cells to introduce a DNA, which has been reported for *Bacillus subtilis* (Duncan, C.H., Wilson, G.A. and Young, F.E., Gene, 1, 153 (1977)) can be employed. In addition to these methods, a method of introducing a recombinant DNA into protoplast- or spheroplast-like recipient cells, which haved been reported to be applicable to *Bacillus subtilis,* actinomycetes, and yeasts (Chang, S. and Choen, S.N., Molec. Gen. Genet., 168, 111 (1979); Bibb, M.J., Ward, J.M. and Hopwood, O.A., Nature, 274,398 (1978); Hinnen, A., Hicks, J.B. and Fink, G.R., Proc. Natl. Sci., USA, 75,1929 (1978)), can be employed

The copy number of the *pgl* gene can also be increased by integrating multiple copies of *pgl* the gene on a chromosomal DNA of a microorganism. In order to integrate multiple copies of the *pgl* gene on a chromosomal DNA of a microorganism, homologous recombination can be performed by targeting a sequence on a chromosomal DNA in multiple copies. As a sequence which exists on a chromosomal DNA in multiple copies, repetitive DNA and inverted repeats existing at an end of a transposon can be used as a sequence in which multiple copies exist on a chromosomal DNA. Alternatively, as disclosed in JP2-109985A, it is also possible to incorporate the *pgl* gene into a transposon, and allow it to be transferred so that multiple copies of the gene are integrated into the chromosomal DNA. Integration of the *pgl* gene into the chromosome can be confirmed by southern hybridization using a probe having a partial sequence of the *pgl* gene.

The bacterium used in the present invention includes one wherein the activity of the protein used in the present invention is enhanced by alteration of a expression control sequence of DNA encoding a protein as defined in (A) or (B) on the chromosome of the bacterium (WO00/18935). The enhancement of gene expression can be achieved by placing the DNA used in the present invention under the control of a more potent promoter instead of the native promoter. For example, the *lac* promoter, *trp* promoter, *trc* promoter, *tac* promoter, PR promoter and so forth are known as strong promoters. The term "native promoter" means a DNA region present in the wild-type organism, located upstream of the open reading frame (ORF) of the gene and having a function of promoting transcription of the gene. Strength of a promoter is defined by the frequency of acts of RNA synthesis initiation. Methods for evaluating the strength of the promoter are described in, for example, Deuschle U., Kammerer W., Gentz R., Bujard H. (Promoters in Escherichia coli: a hierarchy of in vivo strength indicates alternate structures. EMBO J. 1986, 5, 2987-2994). A method for evaluating potency of promoter and examples of potent promoters are disclosed in Goldstein et al. (Prokaryotic promoters in biotechnology. Biotechnol. Annu. Rev., 1995, 1, 105-128).

Enhancing the translation can be achieved by introducing into the DNA used in the present invention a more efficient Ribosome Binding Site(RBS) in place of the native RBS sequence. The RBS sequence is a region upstream of the start codon of the mRNA which interacts with the 16S RNA of ribosome (Shine J. and Dalgarno L., Proc. Natl. Acad. Sci. U S A, 1974, 71, 4, 1342-6). The term "native RBS sequence" means RBS sequence presented in the wild-type organism. The RBS sequence of the gene *10* from phage T7 can be exemplified as an efficient RBS sequence (Olins P.O. et al, Gene, 1988, 73, 227-235).

The bacterium used in the present invention can be obtained by introduction of the aforementioned DNAs into a bacterium inherently having the ability to produce L-amino acids. Alternatively, the bacterium used in present invention can be obtained by imparting the ability to produce L-amino acid to the bacterium already harboring the DNAs.

As a parent strain which is to be enhanced in the activity of the protein used in the present invention, the L-tryptophan-producing bacterium belonging to the genus *Escherichia,* the *E. coli* strains JP4735/pMU3028 (DSM10122) and JP6015/pMU91 (DSM10123) deficient in the tryptophanyl-tRNA synthetase encoded by mutant *trpS* gene (US patent 5,756,345); *E. coli* strain SV164 (pGH5) having *serA* allele free from feedback inhibition by serine (US patent 6,180,373); *E. coli* strains AGX17 (pGX44) (NRRL B-12263) and AGX6(pGX50)aroP (NRRL B-12264) deficient in the enzyme tryptophanase (US patent 4,371,614); *E. coli* strain AGX17/pGX50,pACKG4-pps in which a phosphoenolpyruvate-producing ability is enhanced (WO9708333, US patent 6,319,696) and the like may be used. The inventors of the present invention previously identified that the *yddG* gene encoding a membrane protein, which is not involved in biosynthetic pathway of any L-amino acid, conferred on a microorganism resistance to L-phenylalanine and several amino acid analogues when the wild-type allele of the gene was amplified on a multi-copy vector in the microorganism. Besides, the *yddG* gene can enhance production of L-phenylalanine or L-tryptophan when additional copies are introduced into the cells of the respective producing strain (Russian patent application 2002121670, WO03044192). Therefore, it is desired that the L-tryptophan-producing bacterium be further modified to have enhanced expression of *yddG* open reading frame.

Genes effective for L-tryptophan biosynthesis include genes of the *trpEDCBA* operon, genes of a common pathway for aromatic acids, such as *aroF, aroG, aroH, aroB, aroD, aroE, aroK, aroL, aroA,* and *aroC* genes, genes of L-serine biosynthesis, such as *serA, serB,* and *serC* genes, and the like.

As a parent strain which is to be enhanced in activity of the protein used in the present invention, the phenylalanine-producing bacterium belonging to the genus *Escherichia,* the *E. coli* strain AJ12739 (tyrA::Tn10, tyrR); strain HW1089 (ATCC Accession No. 55371) harboring pheA34 gene (US5,354,672); mutant MWEC101-b strain (KR8903681); strains NRRL B-12141, NRRL B-12145, NRRL B-12146 and NRRL B-12147 (US4,407,952) and the like may be used. The phenylalanine-producing bacterium belonging to the genus *Escherichia* further includes the *E. coli* strain K-12 [W3110 (tyrA)/pPHAB], *E. coli* strain K-12 [W3110 (tyrA)/pPHAD], *E. coli* K-12 [W3110 (tyrA)/pPHATerm] and *E. coli* strain K-12 [W3110 (tyrA)/pBR-aroG4,pACMAB] named as AJ 12604 and the like (European patent EP488424B1).

As a parent strain which is to be enhanced in the activity of the protein used in the present invention, the L-tyrosine-producing bacterium belonging to the genus *Escherichia,* the *E. coli* strains wherein a phosphoenolpyruvate-producing ability or the enzyme of common aromatic pathway is enhanced and the like may also be used (EP0877090A).

The method of the present invention includes a method for producing an L-amino acid, comprising the steps of cultivating the bacterium used in the present invention in a culture medium, to allow the L-amino acid to be produced and accumulated in the culture medium, and collecting the L-amino acid from the culture medium. Also, the method of present invention includes a method for producing L-tryptophan comprising steps of cultivating the bacterium used in the present invention in a culture medium, to allow L-tryptophan to be produced and accumulated in the culture medium, and collecting L-tryptophan from the culture medium. The method of present invention includes a method for producing L-phenylalanine comprising steps of cultivating the bacterium used in the present invention in a culture medium, to allow L-phenylalanine to be produced and accumulated in the culture medium, and collecting L-phenylalanine from the culture medium. The method of present invention further includes a method for producing L-tyrosine, comprising steps of cultivating the bacterium used in the present invention in a culture medium, to allow L-tyrosine to be produced and accumulated in the culture medium, and collecting L-tyrosine from the culture medium.

In the present invention, the cultivation, collection, and purification of L-amino acids, preferably aromatic amino acids such as L-tryptophan, L-phenylalanine, and L-tyrosine from the medium and the like may be performed in a manner similar to conventional fermentation methods wherein an amino acid is produced using a microorganism.

A medium used for culture may be either a synthetic or natural medium, so long as the medium includes a carbon source and a nitrogen source and minerals and, if necessary, appropriate amounts of nutrients which the microorganism requires for growth.

The carbon source includes various carbohydrates such as glucose and sucrose, and various organic acids. Depending on the mode of assimilation of the chosen microorganism, alcohol including ethanol and glycerol may be used.

As the nitrogen source, various ammonium salts such as ammonia and ammonium sulfate, other nitrogen compounds such as amines, a natural nitrogen source such as peptone, soybean-hydrolysate and digested fermentative microorganism may be used.

As minerals, potassium monophosphate, magnesium sulfate, sodium chloride, ferrous sulfate, manganese sulfate, calcium chloride, and the like may be used.

Additional nutrients can be added to the medium, if necessary. For instance, if the microorganism requires tyrosine for growth (tyrosine auxotrophy), a sufficient amount of tyrosine can be added to the medium for cultivation.

The cultivation is performed preferably under aerobic conditions such as a shaking culture, and stirring culture with aeration, at a temperature of 20 to 42 °C, preferably 37 to 40 °C. The pH of the culture is usually between 5 and 9, preferably between 6.5 and 7.2. The pH of the culture can be adjusted with ammonia, calcium carbonate, various acids, various bases, and buffers. Usually, a 1 to 5-day cultivation leads to the accumulation of the target L-amino acid in the liquid medium.

After cultivation, solids such as cells can be removed from the liquid medium by centrifugation or membrane filtration, and then the target L-amino acid can be collected and purified by ion-exchange, concentration and crystallization methods.

### Examples

The present invention will be explained more specifically below with reference to the following non-limiting Examples.

### Example 1. Identification of pgl gene from E. coli and nucleotide sequences comparison.

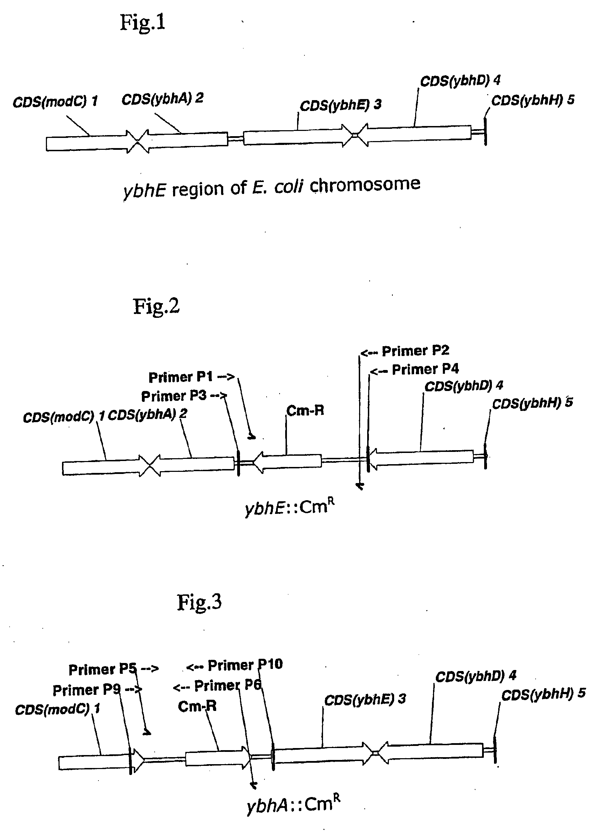

Kupor and Fraenkel mappedpgl mutation between *chlD* (presently known as *modC*) and *bioA* genes on the chromosome of *E. coli* (Kupor, S.R. and Fraenkel, D.G., J. Bacteriol., 100:3, 1296-1301 (1969)). This corresponds to 17.18 and 17.40 minutes of *E, coli* genetic map. In this region, there are eight open reading frames encoding proteins with unknown function. Further, *E*. *coli* Stock Center Database places *pgl* mutation between 17.20 and 17.22 minutes. These coordinates nearly exactly match the coordinates of the *ybhE* opened reading frame (ORF) located between *ybhA* and *ybhD* ORFs (Fig. 1).

BLAST searches performed with YbhE protein encoded by *ybhE* showed that there are many homologs with unknown function in different organisms, such as *Shigella flexmeri* (98.8% similarity), *Salmonella typhi* (92,8% similarity), *Yersinia pestis* (68,4% similarity), several homologs with known function, such as cytochrome D1 heme domain from *Bacillus anthracis* (28% identity), 3-carboxymuconate cyclase from *Pseudomonas fluorescens* predicted by automated computational analysis (28% identity), muconate cycloisomerase from *Trichosporon beigelii* (26% identity), and the one from *Bacillus cereus* mentioned as 6-phosphogluconolactonase in the databanks under accession number NP_833107, but without reference to published experimental work.

Also, three overlaid conserved protein domains were found using NCBI Conserved Domain Search. Two of them belong to conserved protein families with uncharacterized function, and one belongs to 3-carboxymuconate cyclase family.

A BLAST search within *E*. *coli* proteome did not revealed homologs of described 6-phosophogluconolactonases, for example, from *Pseudomonas putida.*

To identify whether the ORF marked as *ybhE* in the *E*. *coli* chromosome is *pgl* gene encoding 6-phosophogluconolactonase, consequent disruption of the *ybhA, ybhE* and *ybhD* ORFs was performed and the obtained mutants were checked for "maltose blue" phenotype (see below).

### Example 2. Disruption of the ybhE ORF. Substitution of the ybhE ORF by the DNA fragment carrying chloramphenicol resistance gene (Cm^{R}).

To disrupt *ybhE* ORF, the DNA fragment carrying chloramphenicol resistance marker (Cm^{R}) encoded by *cat* gene was integrated into the chromosome of the *E. coli* strain BW25113[pKD46] instead of the native *ybhE* ORF by method described by Datsenko K.A. and Wanner B.L. (Proc.Natl.Acad.Sci.USA, 2000, 97, 6640-6645) which is also called as a "Red-mediated integration" and/or "Red-driven integration". The nucleotide sequence of the substituted native region of *ybhE* ORF and the amino acid sequence encoded by the ORF are presented in the Sequence listing (SEQ ID NOs: 1 and 2, respectively). *Escherichia coli* strain BW25113 containing the recombination plasmid pKD46 can be obtained from the *E*. *coli* Genetic Stock Center, Yale University, New Haven, USA, the accession number of which is CGSC7630.

A DNA fragment containing Cm^{R} marker was obtained by PCR using the commercially available plasmid pACYC184 (GenBank/EMBL accession number X06403, "Fermentas", Lithuania) as the template and primers P1 (SEQ ID NO: 3) and P2 (SEQ ID NO: 4). Primer ID contains 36 nucleotides homologous to the 5'-terminus of *ybhE* ORF and primer P2 contains 36 nucleotides homologous to 3'-terminus of *ybhE* ORF. These sequences of *ybhE* gene were introduced into primers P1 and P2 for further integration into the bacterial chromosome.

PCR was provided using the "TermoHybaid PCR Express" amplificator. The reaction mixture (total volume - 50 µl) consisted of 5 µl of 10x PCR-buffer with 15 mM MgCl₂ ("Fermentas", Lithuania), 200 µM each of dNTP, 25 pmol each of the exploited primers and 1 U of Taq-polymerase ("Fermentas", Lithuania). Approximately 5 ng of the plasmid DNA was added in the reaction mixture as a template DNA for the PCR amplification. The temperature profile was the following: initial DNA denaturation for 5 min at 95 °C, followed by 25 cycles of denaturation at 95 °C for 30 sec, annealing at 55 °C for 30 sec, elongation at 72 °C for 30 sec; and the final elongation for 7 min at 72 °C.

Then, the amplified DNA fragment was purified by the agarose gel-electrophoresis, extracted using "GenElute Spin Columns" ("Sigma", USA) and precipitated by ethanol. Nucleotide sequence of the constructed DNA fragment is presented in SEQ ID NO: 5.

The obtained DNA fragment purified as described above was used for electroporation and Red-mediated integration into the bacterial chromosome of the *E. coli* strain BW25113[pKD46]. The recombinant plasmid pKD46 (Datsenko, K.A., Wanner, B.L., Proc.Natl.Acad.Sci.USA, 2000, 97, 6640-6645) with the thermosensitive replicon was used as the donor of the phage λ-derived genes responsible for functioning in the Red-mediated recombination system.

BW25113[pKD46] cells were grown overnight at 30 °C in the liquid LB-medium with addition of ampicillin (100 µg/ml), then diluted 1:100 by the SOB-medium (Yeast extract, 5 g/l; Nacl, 0.5 g/l; Tryptone, 20 g/l; KCI, 2.5 mM; MgCl₂, 10 mM) with addition of ampicillin (100 µg/ml) and L-arabinose (10 mM) (arabinose is used for inducing the plasmid encoding genes of Red system) and grown at 30 °C to reach the optical density of the bacterial culture OD₆₀₀=0.4-0.7. The grown cells from 10 ml of the bacterial culture were washed 3 times by the ice-cold de-ionized water, followed by suspending in 100 µl of the water. 10 µl of DNA fragment (100 ng) dissolved in the de-ionized water was added to the cell suspension. The electroporation was performed by "Bio-Rad" electroporator (USA) (No. 165-2098, version 2-89) according to the manufacturer's instructions. Shocked cells were added to 1-ml of SOC medium (Sambrook et al, "Molecular Cloning A Laboratory Manual, Second Edition", Cold Spring Harbor Laboratory Press (1989)), incubated 2 hours at 37 °C, and then were spread onto L-agar containing 25 µg/ml of chloramphenicol. Colonies grown within 24 h were tested for the presence of Cm^{R} marker instead of native *ybhE* ORF by PCR using primers P3 (SEQ ID NO: 6) and P4 (SEQ ID NO: 7). For this purpose, a freshly isolated colony was suspended in 20µl water and then 1µl of obtained suspension was used for PCR. Temperature profile was the following: initial DNA denaturation for 10 min at 95 °C; then 30 cycles of denaturation at 95 °C for 30 sec, annealing at 55 °C for 30 sec and elongation at 72 °C for 1 min; the final elongation for 7 min at 72 °C. A few Cm^{R} colonies tested contained the desired 1279 bp DNA fragment, confirming the presence of Cm^{R} marker DNA instead of the native *ybhE* ORF. One of the obtained strains was cured from the thermosensitive plasmid pKD46 by culturing at 37 °C and the resulting strain was named *E*. *coli* strain BW25113-Δ*ybhE*.

The structure of the bacterial DNA region with disrupted *ybhE* ORF is shown in Fig. 2.

### Example 3. Disruption of the ybhA and ybhD ORFs. Substitution of the ybhA and ybhD ORFs by the DNA fragments carrying chloramphenicol resistance gene (Cm^{R}).

To disrupt *ybhA* and *ybhD* ORFs, the DNA fragments carrying chloramphenicol resistance marker (Cm^{R}) encoded by *cat* gene were separately integrated in the chromosomes of the *E. coli* strain BW25113[pKD46] instead of the native *ybhA* and *ybhD* ORFs by the method described in Example 2.

To obtain the fragments for electroporation and disruption of *ybhA* and *ybhD* ORFs, two pairs of primers P5 (SEQ ID NO: 8) and P6 (SEQ ID NO: 9), and P7 (SEQ ID NO: 10) and P8 (SEQ ID NO: 11), respectively, were synthesized and used for PCR. Primer P5 contains 36 nucleotides homologous to 3'-terminus of *ybhA* ORF. Primer P6 contains 36 nucleotides homologous to 5'-terminus of *ybhA* ORF. Primer P7 contains 36 nucleotides complementary to the 3'-terminus of *ybhD* ORF. And primer P8 contains 36 nucleotides complementary to 5'-terminus of *ybhD* ORF. These sequences were introduced into primers P5, P6, P7 and P8 for further integration into the bacterial chromosome.

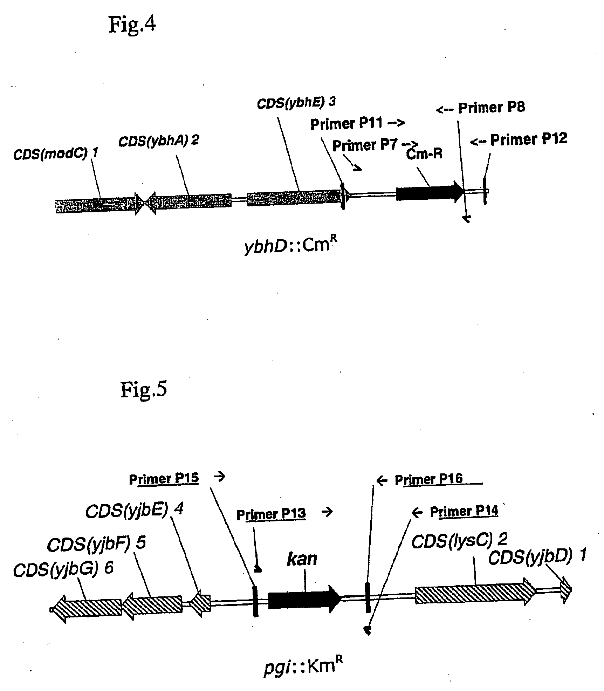

Nucleotide sequences of the constructed DNA fragments are presented in SEQ ID NO: 12 and SEQ ID NO: 13, respectively. Nucleotide sequences of the substituted native regions of *ybhA* and *ybhD* ORFs are presented in GenBank under accession number NC_000913.1 (nucleotide numbers 796836 to 797654 and 798845 to 799777, gi:16128734 and gi:33347481, respectively). The structure of the bacterial DNA regions with disrupted *ybhA* and *ybhD* ORFs are shown in Fig. 3 and Fig. 4, respectively.

After electroporation, corresponding colonies were tested for the presence of Cm^{R} marker by PCR using primers P9 (SEQ ID NO: 14) and P10 (SEQ ID NO: 15) for disruption of *ybhA* ORF and primers P11 (SEQ ID NO: 16) and P12 (SEQ ID NO: 17) for disruption of *ybhD* ORF.

In the first case, a few Cm^{R} colonies tested contained the desired 1424 bp DNA fragment, confirming the presence of Cm^{R} gene instead the native *ybhA* ORF. In the second case, a few Cm^{R} colonies tested contained the desired 1386 bp DNA fragment, confirming the presence of Cm^{R} gene instead the native *ybhD* ORF. In each case, one of the obtained strains was cured from thermosensitive plasmid pKD46 by culturing at 37 °C and the resulting strains were designated *E. coli* strain BW25113-Δ*ybhA* and BW25113-Δ*ybhD*, respectively.

### Example 4. Checking the ybhE⁻, ybhA⁻ and ybhD⁻ mutants for "maltose blue" phenotype.

Each of three obtained mutant strains was tested for "maltose blue" phenotype by the method described by Kupor, S.R. and Fraenkel, D.G. (J. Bacteriol.,100:3, 1296-1301 (1969)). The cultures were patched on plates with M9 minimal media containing 0.8 % of maltose. After 6 hours, incubation plates were flooded with 5ml of solution containing 0.01M I₂ and 0.03M KI, and the patch color was visually scored as "blue" or "not blue".

The obtained strain BW25113-Δ*ybhE* was scored as "blue", while strains BW25113-Δ*ybhA*, BW25113-Δ*ybhD* and BW25113 (as control strain) were "not blue". Example 5. Construction of the double mutant strains carrying pgi and ybhE or ybhD deletions. Comparison of growth of such strains on different carbon sources.

The mutant strain lacking phosphoglucose isomerase (*pgi*⁻) grew slowly on glucose using the oxidative branch of pentose phosphate pathway exclusively. A secondary mutant also lacking phosphogluconolactonase (*pgl*) catalyzing the second step of this branch should grow more slowly due to the spontaneous hydrolysis of 6-phosphogluconolactone to gluconate-6-phosphate only. So, if *ybh*E ORF is really *pgl* gene, the double *pgi*, *ybhE* mutant will grow slower than wild-type strain and *pgi* mutant. To support this suggestion, double *pgi*, *ybhE* mutant was prepared.

The mutation in *pgi* gene was performed by substitution of the native bacterial chromosome region in *E. coli* strain BW25113[pKD46] with the DNA fragment carrying kanamycin resistance gene (Km^{R}) by the method described in Example 2. The nucleotide sequence of the substituted native region of *pgi* gene is presented in GenBank under accession number NC_000913.1 (nucleotide numbers 4231337 to 4232986; gi:16131851).

DNA fragments carrying the Km^{R} gene were obtained by PCR using the commercially available plasmid pUC4KAN (GenBank/EMBL accession number X06404, "Fermentas", Lithuania) as the template and primers P13 (SEQ ID NO: 18) and P14 (SEQ ID NO: 19). Primer P13 contains 36 nucleotides homologous to the 3'-terminus of *pgi* gene and primer P14 contains 36 nucleotides of homologous to the 5'-terminus of *pgi* gene. These sequences from the *pgi* gene were introduced into primers P13 and P14 for further integration into the bacterial chromosome.

PCR was conducted as described in Example 2.

Then, the amplified DNA fragment was concentrated by agarose gel-electrophoresis, extracted from the gel by the centrifugation through "GenElute Spin Columns" ("Sigma", USA) and precipitated by ethanol. The nucleotide sequence of the constructed DNA region is presented in SEQ ID NO: 20.

The obtained DNA fragment purified as described above was used for electroporation and Red-mediated integration into the bacterial chromosome of the *E*. *coli* strain BW25113[pKD46] as described in Example 2, except that cells were spread after electroporation onto L-agar containing 50 µg/ml of kanamycin.

Colonies grown within 24 h were tested for the presence of Km^{R} marker instead of *pgi* gene by PCR using primers P15 (SEQ ID NO: 21) and P16 (SEQ ID NO: 22). For this purpose, a freshly isolated colony was suspended in 20µl water and then 1µl of obtained suspension was used for PCR. PCR conditions were as described in Example 2. A few Km^{R} colonies tested contained the desired 1286 bp DNA fragment confirming the presence of Km^{R} gene instead of *pgi* gene. One of the obtained strains was cured from thermosensitive plasmid pKD46 by culturing at 37 °C and the resulting strain was named *E. coli* strain BW25113-Δ*pgi*.

The structure of the bacterial DNA region with the *pgi* gene deleted is shown on Fig. 5.

The *pgi* deletion was transduced by the method of Fraenkel (J.Bacteriol. 93(1967), 1582-1587) into *E. coli* MG1655 strain followed by selection on plates containing kanamycin. The obtained strain was named MG-Δ*pgi*. Then, mutations in *ybhE* and *ybhD* ORFs were transduced from strains BW25113-Δ*ybhE* and BW25113-Δ*ybhD* described in Examples 2 and 3 into the obtained strain, followed by selection on plates containing chloramphenicol. Obtained strains were designated MG-Δ*pgi-*Δ*ybhE* and MG-Δ*pgi*-Δ*ybhD*, respectively.

These two strains, together with MG1655 and MG1655-Δ*pgi*, were patched on M9 minimal plates with glucose or gluconate as a carbon source. After 24h of incubation the growth of the strains was visually tested. The growth of MG-Δ*pgi-*Δ*ybhE* was worse than for all other strains on the plate with glucose and indistinguishable on the plate with gluconate.

### Example 6. Construction of the plasmid carrying pgl gene from Pseudomonas putida and complementation of the ybhE mutation.

The *pgl* genes from several organisms have been described. Among them is the 6-phosphogluconolactonase from *Pseudomonas putida,* an organism rather closely related to *E. coli.* Several other genes were cloned from *P. putida* into *E. coli* and complementation of the corresponding mutations presented in *E*. *coli* was reported (Ramos-Gonzalez, M.I. and Molin, S., J. Bacteriol., v180, 13, p. 3421,1998).

The *pgl* gene from *P. putida* was cloned using the primers 17 (SEQ ID No. 23) and 18 (SEQ ID No. 24). The primer P17 contains a sequence which is identical to a sequence from 1 to 19 bp of the *pgl* gene from *P. putida.* The primer also contains the ribosome binding site (RBS) of *lacZ* gene from *E. coli* located upstream and a recognition site for the restriction enzyme *Sac*I introduced at the 5'-end thereof. The primer P18 contains a sequence complementary to a sequence from 709 to 729 bp of the *pgl* gene from *P. putida* and a recognition site for restriction enzyme *Eco*RI introduced at the 5'-end thereof.

The chromosomal DNA of *P*. *putida* KT2440 strain TG1 (Bagdasarian, M. & Timmis, K. N. In Current Topics of Microbiology and Immunology, eds. Goebel, W. & Hofschneider,P. H. (Springer, Berlin), pp. 47-67 (1981)) was prepared by a typical method. PCR was carried out on "Perkin Elmer GeneAmp PCR System 2400" under the following conditions: 40 sec. at 95 °C, 40 sec. at 53 °C, 40 sec. at 72 °C, 25 cycles with *Taq* polymerase (Fermentas). The obtained PCR fragment containing the *pgl* gene from *P. putida* with RBS of lacZ gene was treated with *Sac*I and *Eco*RI restrictases and inserted into multicopy vector pUC19 previously treated with the same restrictases. Thus, the plasmid pUC19-pgl was obtained.

Strain BW25113-ΔybhE was transformed by the obtained plasmid pUC19-pgl. The culture was patched on minimal-maltose plates containing 100µg/ml of ampicillin and treated as described above to check the "maltose blue" phenotype. Transformants did not show the "maltose blue" phenotype in the contrast to the control strain BW25113-Δ*ybhE.*

So, the cloned copy of *pgl* gene from *Pseudomonas putida* complements the *ybhE* mutation in *E. coli* once more supporting our hypothesis that *ybhE* ORF is the coding region of *pgl* gene.

### Example 7. Measuring the 6-phosphogluconolactonase activity in ybhE mutant.

The overnight cultures of strains BW25113 and BW25113-Δ*ybhE* were diluted 50 times with minimal M9 media containing glucose. Cells were grown until the optical density of the culture reached OD₅₄₀=1. Extracts were prepared from 3 ml cultures. Cells were washed by physiological solution, resuspended in 400µl of potassium phosphate buffer (pH 7.0) and sonicated. Then, the supernatant fractions obtained after centrifugation were used in the assay without further dilution.

For the measurement of 6-phosphogluconolactonase activity, the method described by Collard, F. et al. (FEBS Letters 459 (1999) 223-226) was used Lactone was prepared extemporaneously by incubating 50 µM glucose-6-phosphate (Sigma, USA) in the presence of 0.2 mM NADP, 25 mM HEPES (pH 7.1), 2 mM MgCl₂ and 1.75 U yeast glucose-6-phosphate dehydrogenase (Sigma, USA) at 30 °C (total volume -1 ml). When the optical density of the reaction mixture at A₃₄₀ reached a plateau, 0.5 U/ml 6-phosphogluconate dehydrogenase (Sigma, USA) along with earlier obtained supernatant fractions to be assayed were added and optical density at A₃₄₀ was further measured for about 10 min. The amount of protein was measured according to method of Bradford, M.M. (Anal. Biochem. 72, 248-254 (1976)). The data obtained are shown in Table 1. Activity is shown in relative units per mg of total protein.

**Table 1.**

| Strain | 6-phosphogluconolactonase activity (for various extract concentrations) |
|---|---|
| BW25113 | 4.0 |
| | 6.1 |
| | 5.4 |
| BW25113-Δ*ybhE* | 0.3 |
| | 0.2 |
| Spontaneous hydrolysis | 0.3 |

As could be seen from the table, 6-phosphogluconolactonase activity in *ybhE* mutant is at least one order of magnitude less than in the "wild-type" strain and is comparable with rate of spontaneous hydrolysis.

### Example 8. Deletion of the zwf-edd-eda operon. Substitution of the zwf-edd-eda genes region by the DNA fragments carrying kanamycin resistance gene (Km^{R}).

In order to obtain the strain with increased YbhE expression we planned the integration of constitutive promoter derived from P*_{tac}* between *ybhE* RBS and its native promoter using Red-mediated integration (see Example 9).

But we failed to provide such chromosome modification of the "wild-type" strain MG1655. We can not explain the toxic effect of enhanced expression of *pgl*(*ybhE*)*,* but we proposed that it is concerned with increased 6-phosphogluconolactonase activity leading to disbalance of Pentose-Phosphate Pathway(PPP) and possible accumulation of some toxic intermediates(or starvation of some essential for cell survival ones). So, we decided to completely turn off the PPP by deletion of *zwf* gene encoding the first enzyme of this pathway.

The deletion of *zwf-edd-eda* operon was performed by the method described in Example 5 for *pgi* gene. The nucleotide sequence of the substituted native regions of *zwf-edd-eda* operon is presented in GenBank under accession number Inc_000913. (nucleotide numbers 1932863 to 1934338, gi:16129805; 1930817 to 1932628, gi:16129804 and 1930139 to 1930780, gi:16129803 for zwf, edd and *eda* genes, respectively). DNA fragments carrying Km^{R} gene were obtained by PCR using primers P19 (SEQ ID NO: 25) and P20 (SEQ ID NO: 26). Primer P19 contains 36 nucleotides complementary to the 3'-terminus of *eda* gene. Primer P20 contains 36 nucleotides complementary to the 5'-terminus of *zwf* gene. The nucleotide sequence of the constructed DNA fragment is presented in SEQ ID NO: 27.

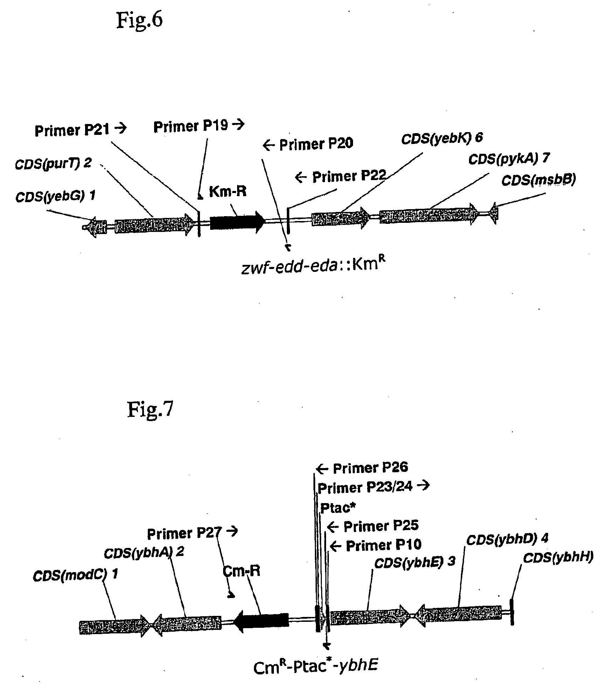

Colonies grown within 24 h were tested for the presence of the Km^{R} marker instead of the *zwf-edd-eda* operon by PCR using primers P21 (SEQ ID NO: 28) and P22 (SEQ ID NO: 29). A few Km^{R} colonies tested contained the desired 1287 bp DNA fragment, confirming the presence of the Km^{R} gene instead of the *zwf-edd-eda* operon. One of the obtained strains was cured from the thermosensitive plasmid pKD46 by culturing at 37 °C and the resulting strain was designated *E. coli* strain BW25113-Δ*zwf-edd-eda.* The structure of the bacterial DNA region having the *zwf-edd-eda* operon deleted is shown in Fig. 6.

### Example 9. Substitution of the native upstream region of ybhE gene located on the E. coli chromosome with a novel regulatory element carrying the synthetic P_{tac}*-promoter.

For further integration of artificial P*_{tac}** promoters of various strength upstream of *pgl* (*ybhE*) gene, retransformation of *E. coli* strain BW25113-Δ*zwf-edd-eda* with pKD46 plasmid was performed. The obtained kanamycin and ampicillin resistant strain was designated *E. coli* strain BW25113-Δ*zwf-edd-eda*[pKD46]. Since the pKD46 plasmid is thermosensitive, further selection of transformants was carried out at 30 °C.

It is a well-established fact that mutants with modified "-35"-region of the promoter, which is recognized by the complex of *E. coli* RNA polymerase with σ⁷⁰, possess significantly changed efficiency of the transcription initiation (WO00/18935): So, among the obtained promoters created on the initial random promoter-like sequence, the promoters with different strength might be obtained. Thus, this general approach could be exploited for fine-tuning of the expression level of the gene of interest. The inventors of the present invention previously obtained the library of modified P*_{tac}* promoters with different strengths (hereinafter such modified P*_{tac}* promoters are marked with an asterisk). These promoters differ in 4 central nucleotides of "-35" region. In the present work, two P*_{tac}** promoters with different strengths were used. Based on the values of the activity of β-galactosidase expressed under the control of the corresponding promoter, those were named as P_{*tac*-10000} (usual P*_{tac}*) and P_{*tac*-3900} (with TTGC). central nucleotides instead of original TGAC).

Then, each of these artificial P*_{tac}** promoters were integrated upstream of the coding region of the *pgl* gene into the chromosome of the *E. coli* strain BW25113-Δ*zwf-edd-eda*[pKD46] by the method described above (see Example 2). In addition, the artificial DNA fragment which has the chloramphenicol resistance gene (Cm^{R}) upstream of the promoter region was integrated (see Fig. 7).

Construction of the above-mentioned artificial DNA fragments integrated into the corresponding region of the bacterial chromosomes was fulfilled in the several steps. For the first step, a DNA fragment, which carried the *Bgl*II restriction site in the upstream region and corresponding P*_{tac}** promoter was obtained by PCR.

The chromosomal DNAs from *E. coli* MG1655 strains having artificial P_{*tac*-3900} promoter and P_{*tac*-10000} promoter integrated into chromosome were used for the PCR as a templates. PCR was provided using primers P23 (SEQ ID NO: 30) and P24 (SEQ ID NO: 31) in case of P_{*tac*-3000} and P_{*tac*-10000} respectively, and primer P25 (SEQ ID NO: 32) in both cases. Primers P23 and P24 contain *Bgl*II *-* restriction site introduced in the 5'-end thereof. Primer P25 contains 11 nucleotides (including RBS) upstream of *pgl* gene and the first 25 nucleotides of *pgl* coding region. The above-mentioned sequences were introduced into primer P25 for further integration into the bacterial chromosome.

PCR was conducted using the amplificatory "TermoHybaid PCR Express PCR System". The reaction mixture (total volume 50 µl) consists of: 5 µl of 10x PCR-buffer with 15 mM MgCl₂ ("Fermentas", Lithuania), 200 µM each of dNTP, 25 pmol each of the exploited primers and 1 u Taq-polymerase ("Fermentas", Lithuania). 0.5 µg of the chromosomal DNA was added in the reaction mixture as a template DNA for the further PCR-driven amplification. The temperature PCR condition were as follows: initial DNA denaturation for 5 min at 95 °C followed by 25 cycles of denaturation at 95 °C for 30 sec, annealing at 53 °C for 30 sec, elongation at 72 °C for 30 sec and the final polymerization for 7 min at 72 °C.

The second stage of construction of the DNA fragment of interest was performed. Cm^{R} gene was amplified by PCR using the commercially available plasmid pACYC184 (GenBank/EMBL accession number X06403, "Fermentas", Lithuania) as the template and primers P26 (SEQ ID NO: 33) and P27 (SEQ ID NO: 34). Primer P26 contains the *Bgl*II-restriction site used for further joining with the earlier obtained DNA fragment carrying P*_{tac}** promoter. Primer P27 contains 46 nucleotides complementary to nucleotides 58 to 12 located upstream of *pgl* (*ybhE*) gene start codon from *E. coli,* necessary for further integration of the fragment into the bacterial chromosome.

Amplified DNA fragments were then concentrated by agarose gel-electrophoresis, extracted from the gel by centrifugation through "GenElute Spin Columns" ("Sigma", USA) and precipitated by ethanol. Then, the two obtained DNA fragments were treated with *Bgl*II restriction endonuclease followed by ligation using T4 DNA ligase (Maniatis T., Fritsch E.F., Sambrook, J.: Molecular Cloning: A Laboratory Manual. 2nd edn. Cold Spring Harbor, NY: Cold Spring Harbor Laboratory Press, 1989).

The ligated product was amplified by PCR using primers P25 and P27. PCR was conducted with a reaction mixture (total volume - 50 µl) consisting of: 5 µl of 10x AccuTaq LA buffer ("Sigma",USA), 200 µM each of dNTP, 25pmol each of the exploited primers and 1 µ, AccuTaq LA polymerase ("Sigma", USA). Approximately 50 ng of ligated DNA product was added in the reaction mixture as a template. The PCR temperature cycles were as follows: initial DNA denaturation for min at 95 °C followed by 25 cycles of denaturation at 95 °C for 30 sec, annealing at 55 °C for 30 sec, elongation at 72 °C for 4 min and the final polymerization for 7 min at 72 °C.

Nucleotide sequences of the constructed DNA regions are presented in SEQ ID NO: 35 and 36 for P_{*tac*-3900} and P_{*tac*-1000} promoters, respectively.

The obtained DNA fragments purified as described above were used for electroporation and Red-mediated integration into the bacterial chromosome of the *E. coli* strain BW25113Δ*zwf-edd-eda*[pKD46] as described in Example 2.

Colonies which grew within 24 h in the medium with chloramphenicol were tested for the presence of Cm^{R} marker upstream of the *pgl* gene by PCR using primers P27 (SEQ ID NO: 34) and P10 (SEQ ID NO: 15). The same colonies were also tested for the presence of P*_{tac}** promoter region upstream of the *pgl* gene by PCR using primers P23 (SEQ ID NO: 30) and P24 (SEQ ID NO: 31) for P_{*tac*-3900} and P_{*tac*-10000}, respectively, and P10 (SEQ ID NO: 15). For this purpose, a freshly isolated colony was suspended in 20µl water and then 1µl of the suspension was used for PCR. PCR conditions were as follows: initial DNA denaturation for 10 min at 95 °C; then 30 cycles of denaturation at 95 °C for 30 sec, annealing at 54 °C for 30 sec and elongation at 72 °C for 1 min; the final polymerization for 7 min at 72 °C. A few Cm^{R} colonies tested contained the desired 1193 bp and 124 bp DNA fragments confirming the presence of whole constructed DNA regions upstream of *pgl* gene and hybrid regulatory element, carrying the P*_{tac}** promoter in *E*. *coli* chromosomes, respectively. In both cases, one of the obtained strains was cured from thermosensitive plasmid pKD46 by culturing at 37 °C and resulting strains were named as *E*. *coli* strain BW25113-P*tac*-3900-*ybhE* and BW25113-P*tac-10000-ybhE.* respectively. The structure of constructed DNA region upstream of the *pgl* gene is shown on Fig. 7.

### Example 10. Measuring the 6-phosphogluconolactonase activity in strains with enhanced expression of pgl gene.

The activity of 6-phosphogluconolactonase from strains BW25113-P*_{tac}*-3900-*ybhE* and BW25113-P*_{tac}*-10000-*ybhE* was measured as described in Example 7. Data obtained are shown in Table 2. The level of spontaneous hydrolysis has been subtracted.

**Table 2.**

| Strain | 6-phosphogluconolactonase activity, relative units |
|---|---|
| BW25113 | 5.6 |
| BW251113-P_{*tac*-3900}-*ybhE* | 21.1 |
| BW25113-P_{*tac*-10000}-*ybhE* | 54.0 |

So, the enhanced expression of *pgl* gene leads to an increase in the 6-phosphogluconolactonase activity.

### Example 11. Effect of enhanced expression of pgl gene on tryptophan production.

The tryptophan-producing *E*. *coli* strain SV164[pMW-P*_{lac}*UV5-*serA5-fruR*, pYDDG2] was used as a parental strain for evaluation of effect of enhanced *pgl* gene expression on tryptophan production. The strain SV164 is described in detail in US patent 6,180,373. The strain SV164[pMW-P_{*lac*UV5-}*serA5-fruR*, pYDDG2] is a derivative of the strain SV164, and additionally containing plasmids pMW-P_{*lac*UV5}-*serA5-fruR* and pYDDG2. Plasmid pMW-P_{*lac*UV5}-*serA5-fruR* carries a mutant serA5 gene encoding the protein, which is free from feedback inhibition by serine (WO2004090125 A2). Amplification of the *serA5* gene is necessary for increasing the amount of serine, which is the precursor of L-tryptophan (US patent 6,180,373). Plasmid pYDDG2 is constructed on the basis of pAYCTER3 vector (WO03/044192) and contains the *yddG* gene encoding transmembrane protein (putative exporter) useful for L-tryptophan production. The pAYCTER3 vector is a derivative of a pAYC32, which is a moderate copy number and very stable vector constructed on the basis of plasmid RSF1010, and harboring a marker for streptomycin resistance (Christoserdov A. Y., Tsygankov Y. D, Broad-host range vectors derived from a RSF 1010 Tnl plasmid, Plasmid, 1986, v. 16, pp. 161-167). The pAYCTER3 vector was obtained by introduction of the polylinker from pUC19 plasmid and the strong terminator *rrnB* into pAYC32 plasmid instead of its promoter.

To test the effect on tryptophan production of enhanced expression of *pgl* gene which is under the control of P*_{tac}** promoters, the DNA fragments from the chromosome of the above-mentioned *E. coli* strains BW25113-P_{*tac*-3900}-*yb-hE* and BW25113-P_{*tac*-10000}-*ybhE* were transferred to a tryptophan-producing *E. coli* strain SV164[pMW-P_{*lac*UV5}-*serA5-fruR*] by P1 transduction (Miller, J.H. (1972) Experiments in Molecular Genetics, Cold Spring Harbor Lab. Press, Plainview, NY). Then, plasmid pYDDG2 was introduced into both the SV164[pMW-P_{*lac*UV5}-*serA5-fruR*] strain and the resulted transductants.

The SV164[pMW-P_{*lac*UV5}-*serA5-fruR*, pYDDG2], SV164-P_{*tac*-3900-*ybhE*}[pMW-P_{lacUV5}-*serA5-fruR*, pYDDG2] and SV164-P_{*tac*-10000}-*ybhE*[pMW-P_{lacUV5}-*serA5-fruR*, pYDDG2] strains were cultivated overnight with shaking at 37 °C in 3 ml of nutrient broth supplemented with 100 µg/ml of ampicillin and 50 µg/ml of streptomycin. 0.3 ml of the obtained cultures were inoculated into 3 ml of a fermentation medium containing said antibiotics in 20 x 200 mm test tubes, and cultivated at 37 °C for 40 hours with a rotary shaker at 250 rpm.

The composition of the fermentation medium is presented in Table 3.

**Table 3.**

| Sections | Component | Final concentration |
|---|---|---|
| A | Glucose/sucrose | 40 g/L |
| | MgSO₄•7H₂O | 0.3 g/L |
| | MnSO₄•5H₂O | 5 mg/L |
| | Mameno | 0,058 g/L of Total Nitrogen |
| | (NH₄)₂SO₄ | 15 g/L |
| | KH₂PO₄ | 0,268 g/L |
| | NaCl | 0.143 g/L |
| | L-Methionine | 0,086 g/L |
| | L-Phenylalanine | 0,286 g/L |
| | L-Tyrosine | 0,286 g/L |
| | KCl | 0,286 g/L |
| | FeSO₄•7H₂O | 5 mg/L |
| | Sodium citrate | 667 mg/L |
| | CaCl₂•2H₂O | 4,29 mg/L |
| | Sterilize at 116 °C for 30 min. | |
| B | Thiamine HCl | 2,5 mg/L |
| | Na₂MoO₄•2H₂O | 0,15 mg/L |
| | H₃BO₃ | 2,5 mg/L |
| | CoCl2•6H₂O | 0,7 mg/L |
| | CuSO₄• 5H₂O | 0,25 mg/L |
| | ZnSO₄ •7 H₂O | 0,3 mg/L |
| | Sterilize at 110 °C for 30 min. | |
| C | Pyridoxin | 45 mg/L |
| | Filtration | |
| Section A had pH 7.1 adjusted by NH₄OH. Each section was sterilized separately. | | |

After the cultivation, the amount of L-tryptophan which accumulated in the medium was determined by TLC. 10 x 15 cm TLC plates coated with 0.11 mm layers of Sorbfil silica gel without fluorescent indicator (Stock Company Sorbpolymer, Krasnodar, Russia) were used. Sorbfil plates were developed with a mobile phase: propan-2-ol : ethylacetate : 25% aqueous ammonia : water = 16 : 16 : 3 : 9 (v/v). A solution (2%) of ninhydrin in acetone was used as a visualizing reagent. Obtained data are presented in the Table 4.

**Table 4**

| Strain | OD₆₀₀ | Amount of tryptophan, g/l |
|---|---|---|
| SV164[pMW-P_{*lac*UV5}-*serA5-fruR*, pYDDG2] | 7.5 | 4.20 |
| SV164-P_{tac-3900-*ybhE*}[PMW-P_{*lac*UV5}-*serA5-fruR*, pYDDG2] | 7.5 | 4.61 |
| SV164-Pt_{*tac*-10000-*ybhE*}[PMW-P_{lacUV5}-*serA5-fruR*, pYDDG2] | 7.5 | 4.92 |

As it can be seen from Table 4, the enhancement of *pgl* gene expression improved tryptophan productivity of the SV164[pMW-P_{*lac*UV5}-*serA5-fruR*, pYDDG2] strain.

### Example 12. Purification of His-tagged YbhE protein and determination of its 6-PGL (6-phosphogluconolactonase) activity.

All results previously described in Examples 1-7 serve as indirect indications that *ybhE* ORF is the *pgl* gene that encodes the functionally active 6-PGL in *E. coli.* On the other hand, it is possible that the *ybhE* ORF encodes, for instance, a positive regulator of expression of another unknown gene that, in turn, encodes 6-PGL. So, a final conclusion concerning the nature of *ybhE* ORF can be made only by direct determination of the biological activity of its protein product.

For this purpose, *ybhE* ORF was overexpressed by exploiting the T7 expression system, including *E. coli* BL21(DE3) as the recipient strain carrying T7 RNA polymerase gene in the chromosome, and pET-22b(+) vector plasmid with T7 late promoter and efficient RBS of T7 gene 10. For the convenience of the following protein purification 6 His codons were inserted at 5'-end of *ybhE* ORF, exactly after ATG initiation codon.

To clone the *ybhE* ORF with a His-Tag sequence in the T7 expression system, PCR was conducted with primers P28 (SEQ ID NO: 37) and P29 (SEQ ID NO: 38) using chromosomal DNA of the *E. coli* strain MG1655 as the template. Primer P28 contains a *Nde*I restriction site, and an ATG-codon linked to 6 additional histidine codons before the second codon of the ybhE ORF. Primer P29 contains a *Bam*HI restriction site at its 5'-end for further cloning. The amplified DNA fragment was isolated, treated with *Nde*I and *Bam*HI restrictases, and ligated into a pET-22b(+) plasmid which had been treated with the same restrictases. Construction of the obtained pET-HT-ybhE plasmid was verified by sequencing.

Then, BL21(DE3) cells carrying the T7 RNA polymerase gene under the control of a lactose promoter in their chromosome were transformed with a pET-HT-ybhE plasmid. The overnight culture from a single colony was diluted 50 times with LB and grown to OD₆₀₀∼1.0 followed by addition of IPTG (1 mM) for inducing of T7 RNA polymerase-driven expression of *ybhE* ORF in the recombinant plasmid. After 2 hours of incubation, cells were collected from 20 ml. Cell extracts were prepared by sonication in the buffer containing 20mM Tris-HCl, pH 8.0 and 2 mM PMSF. Then, probes were centrifuged for 20 min at 16,000 x g and 4 °C followed by purification of His-tagged protein from the supernatant using HiTrap Chelating HP Columns (Amersham Bioscience) as recommended by producer.

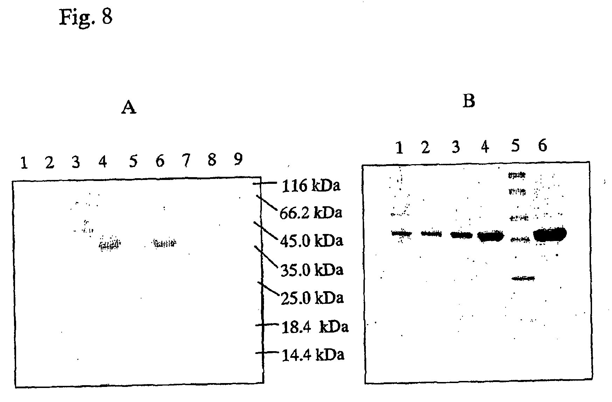

Two hours after induction of the T7 expression system in logarithmic culture, the accumulation of the protein with the electrophoretic mobility corresponding to His-tagged YbhE (protein with MW » 37 KDa) was observed. The amount of the protein was about 15% of total cellular polypeptides. The protein was observed mostly in a soluble phase (see, Figure 8A).

The obtained His₆-YbhE protein was purified using a Ni-NTA column. Determination of the level of the recombinant protein synthesis and control of the purification process were provided by SDS-PAGE electrophoresis according to method described by Laemmli U.K. (Nature, 227, 680-685 (1970)). As can be seen in Fig.8B, the purity of the obtained protein is more than 90%, and it exhibited 6-PGL activity in the standard lactonase test (Collard, F. et al, FEBS Letters, 459, 223-226 (1999)); Example 7). It is interesting to note that the determined specific 6-PGL activity for the purified His₆-YbhE (780 U/mg) is very close to the earlier reported activity of the similarly His₆-tagged human 6-PGL (710 U/mg) (Collard, F. et al, FEBS Letters, 459, 223-226 (1999)).

Thus, it can be concluded that the *ybhE* ORF from *E. coli* with unknown function is indeed the *pgl* gene encoding 6-PGL.

### Example 13. Effect of enhanced expression of pgl gene on phenylalanine production.

The phenylalanine-producing *E.coli* strain AJ12739 was used as a parental strain for evaluating the effect of enhanced *pgl* gene expression on tryptophan production. The strain AJ12739 was deposited in the Russian National Collection of Industrial Microorganisms (VKPM) (Russia, 113545 Moscow, 1^{st} Dorozhny proezd, 1) on November 6, 2001 under accession number VKPM B-8197.

Chromosomal DNA fragments from BW25113-P_{*tac*-3900}-*ybhE* and BW25113-P_{*tac*-10000}-*ybhE* strains were transfered into the phenylalanine-producing strain AJ12739 by P1 transduction, resulting in the AJ12739 *P*_{*tac*-3900}-*ybhE* and AJ12739 P_{*tac*-10000} strains, respectively. These strains were each cultivated at 37 °C for 18 hours in a nutrient broth with 25 mg/l chloramphenicol, and 0.3 ml of the obtained culture was inoculated into 3 ml of a fermentation medium containing 25 mg/l chloramphenicol in a 20 x 200 mm test tube, and cultivated at 34 °C for 24 hours with a rotary shaker. After the cultivation, the amount of phenylalanine which had accumulated in the medium was determined by TLC. 10 x 15 cm TLC plates coated with 0.11 mm layers of Sorbfil silica gel without fluorescent indicator (Stock Company Sorbpolymer, Krasnodar, Russia) were used. Sorbfil plates were developed with a mobile phase: propan-2-ol : ethylacetate : 25% aqueous ammonia : water = 40 : 40 : 7: 16 (v/v). A solution (2%) of ninhydrin in acetone was used as a visualizing reagent.

The composition of the fermentation medium (g/l)

| | |
|---|---|
| Glucose | 40.0 |
| (Nm)₂SO₄ | 16.0 |
| K₂HPO₄ | 0.1 |
| MgSO₄•7H₂O | 1.0 |
| FeSO₄•7H₂O | 0.01 |
| MnSO₄•5H₂O | 0.01 |
| Thiamine HCl | 0.0002 |
| Yeast extract | 2.0 |
| Tyrosine | 0.125 |
| CaCO₃ | 20.0 |

Glucose and magnesium sulfate are sterilized separately. CaCO₃ dry-heat sterilized at 180 °C for 2 h. pH is adjusted to 7.0. Antibiotic is introduced into the medium after sterilization. The results are presented in Table 5.

**Table 5**

| *E. coli* strain | OD₆₀₀ | Amount of phenylalanine, g/l |
|---|---|---|
| AJ12739 | 18.2 ± 0.1 | 0.65 ± 0.4 |
| AJ12739 *P_{tac-3900}-ybhE* | 17.0 ± 0.3 | 0.9 ± 0.1 |
| AJ12739 P*_{tac-10000}-ybhE* | 16.3 ± 0.2 | 1.3 ± 0.1 |

It can be seen from Table 5 that the enhanced expression of *pgl* gene improved phenylalanine production of the AJ12739 strain.

### Industrial Applicability

According to the present invention, production of L-amino acids such as L-Tryptophan, L-phenylalanine, and L-tyrosine can be enhanced.

## Claims

1. A method for producing an L-amino acid comprising cultivating an L-amino acid-producing bacterium belonging to the Enterobacteriaceae family in a culture medium, and collecting said L-amino acid from said culture medium, wherein the bacterium has been modified to increase expression of a gene encoding 6-phosphogluconolactonase, wherein the 6-phosphogluconolactonase is a protein selected from the group consisting of:
(A) a protein comprising the amino acid sequence shown in SEQ ID NO:2, and
(B) a protein comprising an amino acid sequence which includes deletion, substitution, insertion or addition of one to 20 amino acids in the amino acid sequence shown in SEQ ID NO:2, and which has an activity of 6-phosphogluconolactonase,
wherein said expression of the gene is increased by increasing a copy number of said gene, by modifying an expression regulatory sequence of said gene, by substituting a native promoter of said gene with a more potent promoter, or by substituting a native Shine-Dalgarno (SD) sequence of said gene with a more efficient SD sequence.

2. A method according to claim 1, wherein said 6-phosphogluconolactonase gene is selected from the group consisting of:
(a) a DNA comprising a nucleotide sequence of the nucleotides 1 to 993 in SEQ ID NO:1; and
(b) a DNA which is hybridizable with a nucleotide sequence of the nucleotides 1 to 993 in SEQ ID NO:1 under stringent conditions and encodes a protein having an activity of 6-phosphogluconolactonase, and wherein said stringent conditions comprise washing for 15 minutes at 60 °C at a salt concentration corresponding to 1 x SSC and 0.1 % SDS.

3. The method according to claim 1 or 2, wherein the bacterium is selected from the genus selected from the group consisting of Escherichia, Enterobacter, Erwinia, Klebsiella, Pantoea, Providencia, Salmonella, Serratia, Shigella, and Morganella.

4. The method according to any one of claims 1 to 3, wherein said bacterium is further modified to have enhanced expression of a yddG open reading frame, wherein said expression of the yddG gene is enhanced by amplifying said gene on a multi-copy vector.

5. The method according to any one of claims 1 to 4, wherein said L-amino acid is an aromatic L-amino acid selected from the group consisting of L-tryptophan, L-phenylalanine, and L-tyrosine.

## Patentansprüche

1. Verfahren zum Herstellen einer L-Aminosäure, welches das Kultivieren eines L-Aminosäure produzierenden Bakteriums der Familie Enterobacteriaceae in einem Kulturmedium und das Gewinnen der L-Aminosäure aus dem Kulturmedium umfasst, wobei das Bakterium modifiziert worden ist, um die Expression eines für 6-Phosphogluconolactonase kodierenden Gens zu erhöhen, wobei die 6-Phosphogluconolactonase ein Protein ist, das aus der folgenden Gruppe ausgewählt ist:
(A) ein Protein, das die in SEQ ID Nr. 2 gezeigte Aminosäuresequenz umfasst; und
(B) ein Protein, das eine Aminosäuresequenz umfasst, welche Deletion, Substitution, Insertion oder Addition von einer bis 20 Aminosäuren in der in SEQ ID Nr:2 gezeigten Aminosäuresequenz umfasst, und das eine Aktivität von 6-Phosphogluconolactonase hat,
wobei die Expression des Gens durch Erhöhen einer Kopienzahl des Gens, durch Modifizieren einer Expressionsregulationssequenz des Gens, durch Substituieren eines nativen Promotors des Gens durch einen stärkeren Promotor oder durch Substituieren einer nativen Shine-Dalgano (SD)-Sequenz des Gens mit einer effizienteren SD-Sequenz erhöht ist.

2. Verfahren nach Anspruch 1, wobei das 6-Phosphogluconolactonase-Gen aus der folgenden Gruppe ausgewählt ist:
(a) eine DNA, die eine Nukleotidsequenz der Nukleotide 1 bis 993 in SEQ ID Nr:1 umfasst; und
(b) eine DNA, die mit einer Nukleotidsequenz der Nukleotide 1 bis 993 in SEQ ID Nr:1 unter stringenten Bedingungen hybridisierbar ist und für ein Protein mit einer Aktivität von 6-Phosphogluconolactonase kodiert, wobei die stringenten Bedingungen das Waschen während 15 Minuten bei 60 °C bei einer 1 x SSC und 0,1 % SDS entsprechenden Salzkonzentration umfassen.

3. Verfahren nach Anspruch 1 oder 2, wobei das Bakterium aus der Gattung ausgewählt ist, die aus der Gruppe ausgewählt ist, die aus Escherichia, Enterobacter, Erwinia, Klebsiella, Pantoea, Providencia, Salmonella, Serratia, Shigella und Morganella besteht.

4. Verfahren nach einem der Ansprüche 1 bis 3, wobei das Bakterium außerdem modifiziert ist, um eine erhöhte Expression eines offenen Leserahmens von yddG aufzuweisen, wobei die Expression des yddG-Gens durch Amplifizieren des Gens auf einem Mehrkopienvektor erhöht ist.

5. Verfahren nach einem der Ansprüche 1 bis 4, wobei die L-Aminosäure eine aromatische L-Aminosäure ist, die aus der Gruppe ausgewählt ist, die aus L-Tryptophan, L-Phenylalanin und L-Tyrosin besteht.

## Revendications

1. Procédé de production d'un acide L-aminé, comprenant l'étape consistant à cultiver une bactérie produisant un acide L-aminé appartenant à la famille des *Enterobacteriaceae* dans un milieu de culture, et à collecter ledit acide L-aminé à partir dudit milieu de culture, où la bactérie a été modifiée pour augmenter l'expression d'un gène codant pour la 6-phosphogluconolactonase, où la 6-phosphogluconolactonase est une protéine sélectionnée dans le groupe consistant en :
(A) une protéine comprenant la séquence d'acides aminés montrée dans SEQ ID NO: 2 ; et
(B) une protéine comprenant une séquence d'acides aminés qui comprend une délétion, une substitution, une insertion ou une addition de un à 20 acides aminés dans la séquence d'acides aminés montrée dans SEQ ID NO: 2, et qui possède une activité de 6-phosphogluconolactonase,
où ladite expression du gène est augmentée en augmentant le nombre de copies dudit gène, en modifiant une séquence régulatrice de l'expression dudit gène, en substituant un promoteur natif dudit gène par un promoteur plus puissant, ou en substituant une séquence Shine-Dalgarno (SD) native dudit gène par une séquence SD plus efficace.

2. Procédé selon la revendication 1, dans lequel ledit gène de la 6-phosphogluconolactonase est sélectionné dans le groupe consistant en :
(a) un ADN comprenant une séquence de nucléotides des nucléotides 1 à 993 dans SEQ ID NO: 1 ; et
(b) un ADN qui peut s'hybrider avec une séquence de nucléotides des nucléotides 1 à 993 dans SEQ ID NO: 1 dans des conditions stringentes, et qui code pour une protéine ayant une activité de 6-phosphogluconolactonase, et où lesdites conditions stringentes comprennent un lavage pendant 15 minutes à 60 °C à une concentration en sel correspondant à du SSC 1 x et du SDS à 0,1 %.

3. Procédé selon la revendication 1 ou 2, dans lequel la bactérie est sélectionnée à partir du genre sélectionné dans le groupe consistant en *Escherichia, Enterobacter, Erwinia, Klebsiella, Pantoea, Providencia, Salmonella, Serratia, Shigella,* et *Morganella.*

4. Procédé selon l'une quelconque des revendications 1 à 3, dans lequel ladite bactérie est en outre modifiée pour présenter une expression améliorée d'un cadre de lecture ouvert *yddG,* où ladite expression du gène *yddG* est améliorée en amplifiant ledit gène sur un vecteur à copies multiples.

5. Procédé selon l'une quelconque des revendications 1 à 4, dans lequel ledit acide L-aminé est un acide L-aminé aromatique sélectionné dans le groupe consistant en le L-tryptophane, la L-phénylalanine, et la L-tyrosine.
